# EUROPÄISCHE PATENTSCHRIFT

(11) **EP 0 148 501 B1**
(45) Veröffentlichungstag und Bekanntmachung des Hinweises auf die Patenterteilung: **11.05.1988**
(21) Anmeldenummer: 84116313.2
(22) Anmeldetag: 27.12.1984
(51) Int. Cl.: C07D 285/12, A01N 43/82, C07C 161/00

(54) **5-Halogenalkyl-1,3,4-thiadiazol-2-yloxyacetamide**
5-Haloalkyl-1,3,4-thiadiazol-2-yloxyamides
5-Halogénoalkyl-1,3,4-thiadiazol-2-yloxyamides

(30) Priorität: 04.01.1984 DE 3400168
(43) Veröffentlichungstag der Anmeldung: 17.07.1985
(73) Patentinhaber: BAYER AG, 51368 Leverkusen (DE)
(72) Erfinder: Förster, Heinz, Dr., D-5600 Wuppertal 1 (DE); Diehr, Hans-Joachim, Dr., D-5600 Wuppertal 11 (DE); Maurer, Fritz, Dr., D-5600 Wuppertal 1 (DE); Klauke. Erich, Dr., D-5068 Odenthal (DE); Eue, Ludwig, Dr., D-5090 Leverkusen 1 (DE); Santel, Hans-Joachim, Dr., D-5000 Köln 80 (DE); Schmidt, Robert R., Dr., D-5060 Bergisch-Gladbach 2 (DE); Reinecke, Paul, Dr., D-5090 Leverkusen 3 (DE); Hänssler, Gerd, Dr., D-5090 Leverkusen 1 (DE)

(56) Entgegenhaltungen:
- EP-A- 0 018 497
- EP-A- 0 094 541
- DE-A- 2 526 308

## Beschreibung

Die Erfindung betrifft neue 5-Halogenalkyl-1,3,4-thiadiazol-2-yloxyacetamide, mehrere Verfahren zu ihrer Herstellung und ihre Verwendung als Herbizide und Fungizide.

Es ist bereits bekannt, dass 5-Trifluormethyl-1,3,4-thiadiazol-2-yloxyacetamide, wie beispielsweise das N-Ethyl-N-(3-methylphenyl)-5-trifluormethyl-1,3,4-thiadiazol-2-yloxyacetamid oder das N-Benzyl-N-methyl-5-trifluormethyl-1,3,4-thiadiazol-2-yloxyacetamid oder das N-Methyl-N-phenyl- 5-trifluormethyl- 1,3,4-thiadiazol-2-yloxyacetamid, herbizide Eigenschaften besitzen (vgl. DE-OS 30 04 326 und 32 18 482).

Deren herbizide Wirksamkeit gegenüber Schadpflanzen ebenso wie ihre Verträglichkeit gegenüber wichtigen Kulturpflanzen ist jedoch nicht immer in allen Anwendungsbereichen völlig zufriedenstellend. Über eine fungizide Wirkung dieser Substanzklasse ist nichts bekannt.

Es wurden neue 5-Halogenalkyl-1,3,4-thiadiazol-2-yloxyacetamide der allgemeinen Formel (I) gefunden, 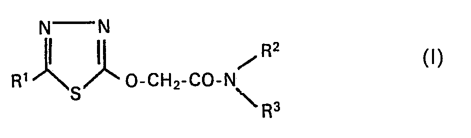 in welcher
R¹ für geradkettiges oder verzweigtes Halogenalkyl mit bis zu 8 Kohlenstoffatomen und bis zu 17 Halogenatomen, mit Ausnahme des Trifluormethylrestes, steht und
R² und R³ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegevenenfalls einfach oder mehrfach, gleich oder verschieden substituiertes Cycloalkyl oder Cycloalkenyl mit jeweils 3 bis 7 Kohlenstoffatomen (wobei als Substituenten insbesondere Alkylreste mit 1 bis 4 Kohlenstoffatomen infrage kommen), für jeweils geradkettiges oder verzweigtes Alkoxy, Alkoxyalkylenoxy und Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten infrage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen (insbesondere Fluor, Chlor und Brom), sowie Nitro, oder
R² und R³ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen, der bis zu 2 weitere Heteroatome, insbesondere Stickstoff und Sauerstoff enthalten kann, wobei als Substituenten infrage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

Weiterhin wurde gefunden, dass man die neuen 5-Halogenalkyl-1,3_{;}4-thiadiazol-2-yloxyacet- amide der allgemeinen Formel (I) erhält, wenn man
(a) 5-Halogenalkyl-1,3,4-thiadiazol-Derivate der Formel (11), 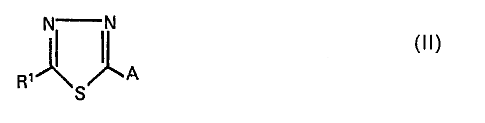 in welcher
   R¹ die oben angegebene Bedeutung hat und
   A für eine elektronenanziehende Abgangsgruppe steht,

   mit Hydroxyacetamiden der Formel (111), 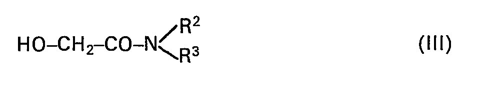 in welcher
   R2 und R³ die oben angegebene Bedeutung haben, gegebenenfalls in Gegenwart eines Verdünnungsmittels und gegebenenfalls in Gegenwart eines Säurebindemittels sowie gegebenenfalls in Gegenwart eines Phasentransferkatalysators umsetzt, oder wenn man
(b) Hydrazin- thiocarbonsäure- O-carbamoylmethylester der Formel (IV), 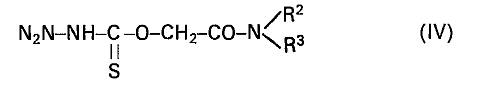 in welcher
   R² und R³ die oben angegebene Bedeutung haben, mit Halogencarbonsäureanhydriden der Formel (V), 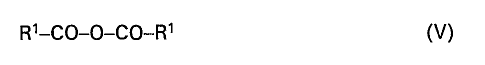
   in welcher
   R¹ die oben angegebene Bedeutung hat, gegebenenfalls in Gegenwart eines Verdünnungsmittels umsetzt, oder wenn man
(c) N'-Acyl- hydrazin- N-thiocarbonsäure-O-carbamoylmethylester der Formel (VI), 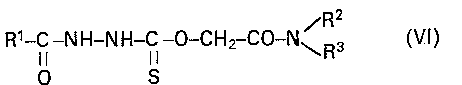 in welcher
   R¹, R² und R³ die oben angegebene Bedeutung haben, mit konzentrierten Mineralsäuren cyclisiert.

Schliesslich wurde gefunden, dass die neuen 5-Halogenalkyl- 1,3,4-thiadiazol- 2-yloxyacetamide der allgemeinen Formel (I) herbizide, insbesondere selektiv herbizide sowie fungizide Eigenschaften besitzen.

Überraschenderweise besitzen die neuen 5-Halogenalkyl-1,3,4-thiadiazol-2-yloxyacet- amide der Formel (I) neben einer verbesserten herbiziden Potenz gegenüber Schadpflanzen auch eine höhere Verträglichkeit gegenüber wichtigen Kulturpflanzen als die aus dem Stand der Technik bekannten 5-Trifluormethyl-1,3,4-thiadiazol-2-yloxyacetamide, welche chemisch und wirkungsmässig naheliegende Verbindungen sind.

Die erfindungsgemässen 5-Halogenalkyl-1,3,4-thiadiazol-2-yloxyacetamide sind durch die Formel (I) allgemein definiert.

Bevorzugt sind Verbindungen der Formel (1), in welchen

R¹ für Chlormethyl, Dichlormethyl, Trichlormethyl, Fluormethyl, Difluormethyl, Chlorfluormethyl, Chlordifluormethyl, Dichlorfluormethyl, 1-Chlorethyl, 2-Chlorethyl, 1,2-Dichlorethyl, 2,2,2-Trichlorethyl, Pentachlorethyl, Pentafluorethyl, 1-Bromethyl, 2-Bromethyl, 2,2,2-Tribromethyl, 1-Chlorpropyl, 2-Chlorpropyl, 3-Chlorpropyl, 1,2-Dichlorpropyl, 1,2,3-Trichlorpropyl, Heptafluorpropyl, Nonafluorbutyl, Dichlorbutyl, Difluorbutyl, Difluorchlorbutyl oder Dichlorfluorbutyl steht und R² und R³ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor, Nitro, oder

R² und R³ zusammen mit dem Stickstoffatom, an das sie gebunden sind, für die gegebenenfalls ein- bis dreifach, gleich oder verschieden substituierten Heterocyclen 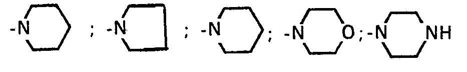 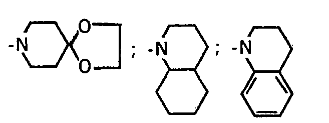 stehen, wobei als Substituenten besonders bevorzugt sind: Methyl, Ethyl und Phenyl.

Im einzelnen seien ausser den bei den Herstellungsbeispielen genannten Verbindungen die folgenden Verbindungen der allgemeinen Formel (I) genannt:

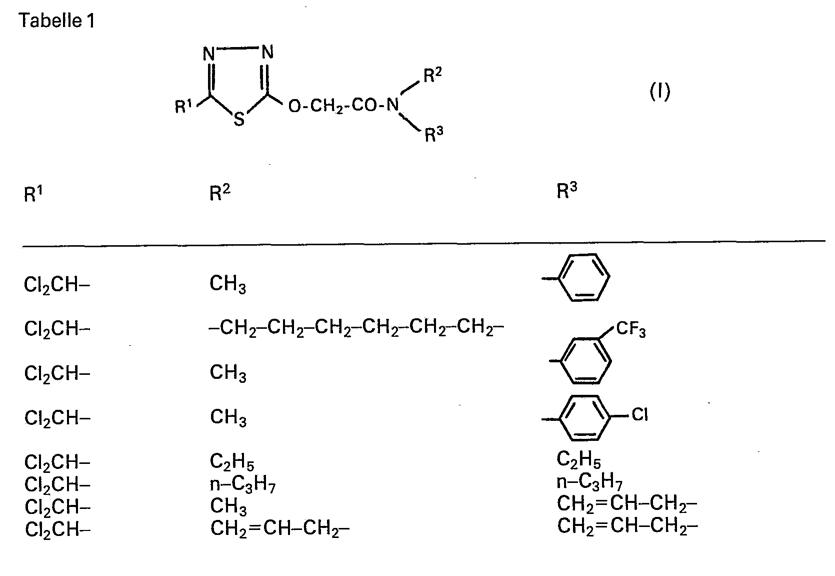

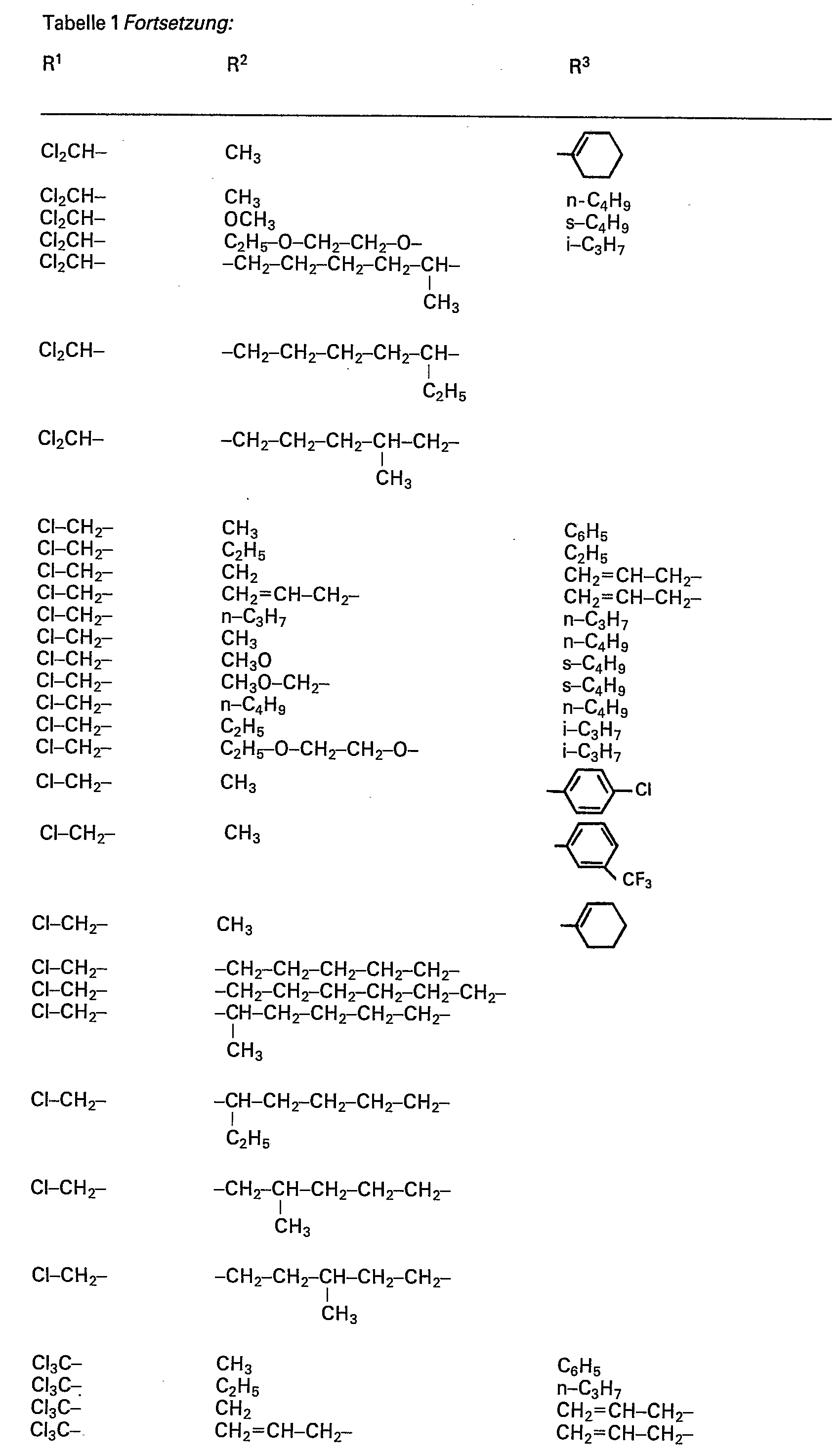

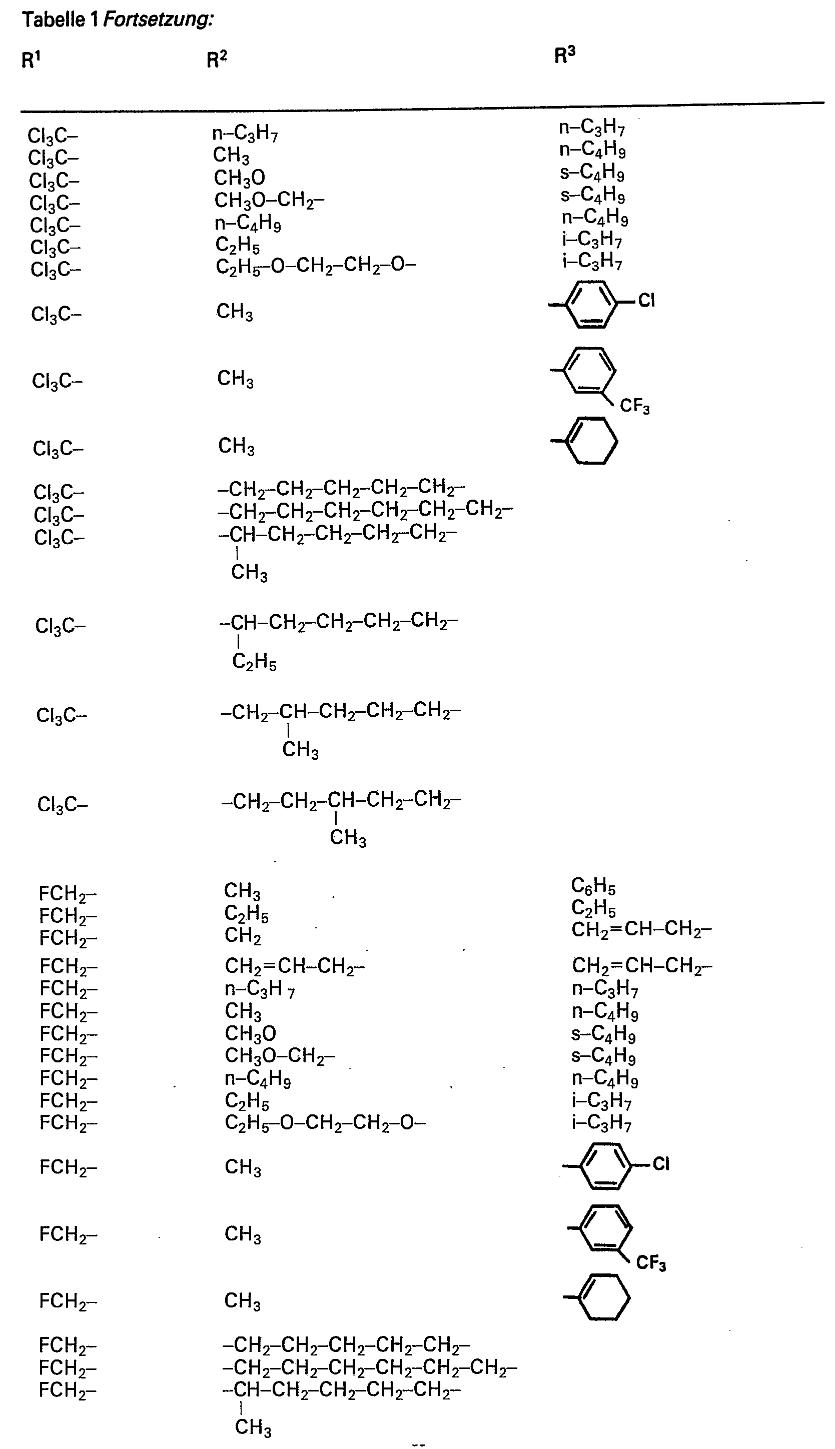

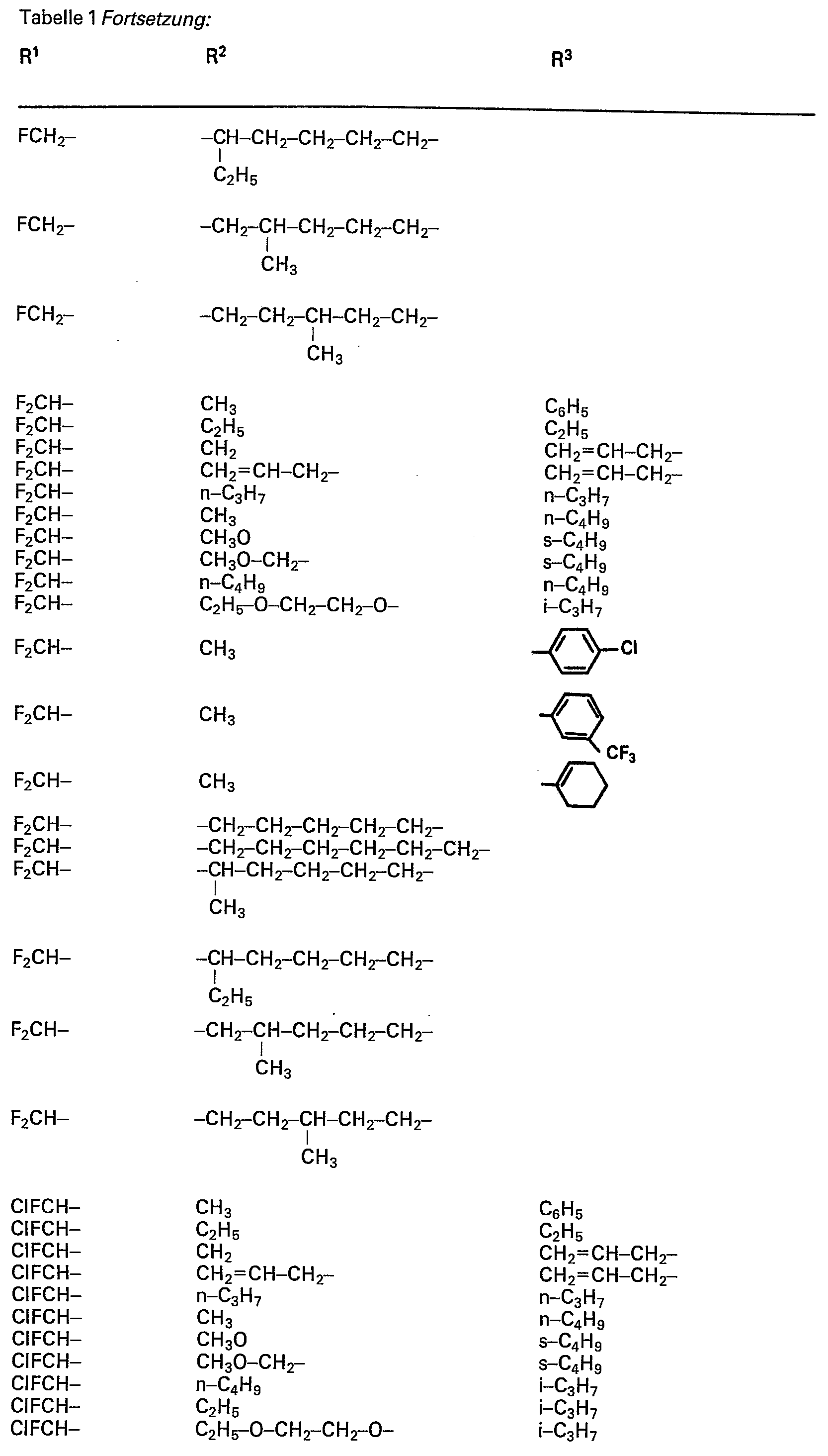

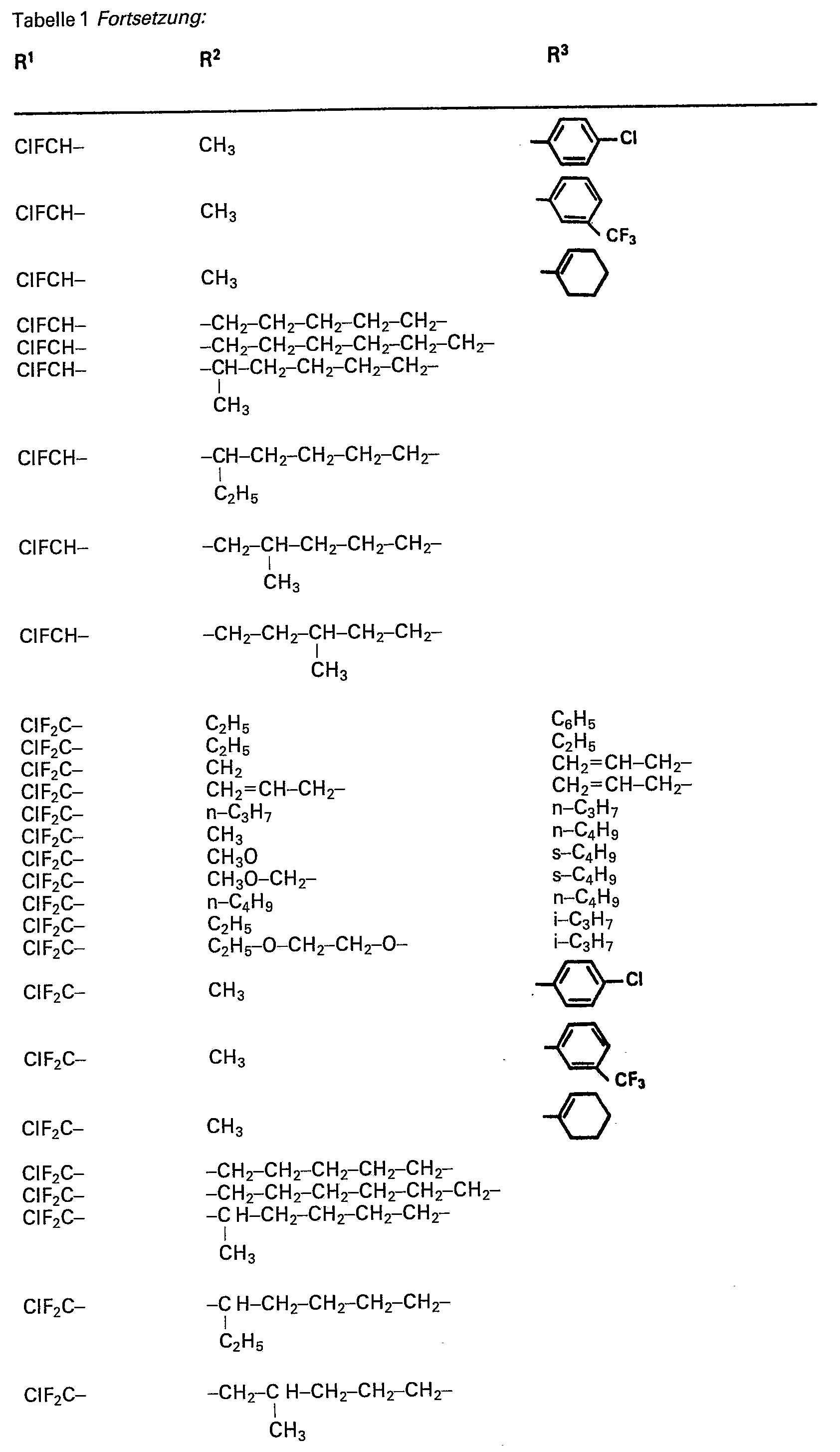

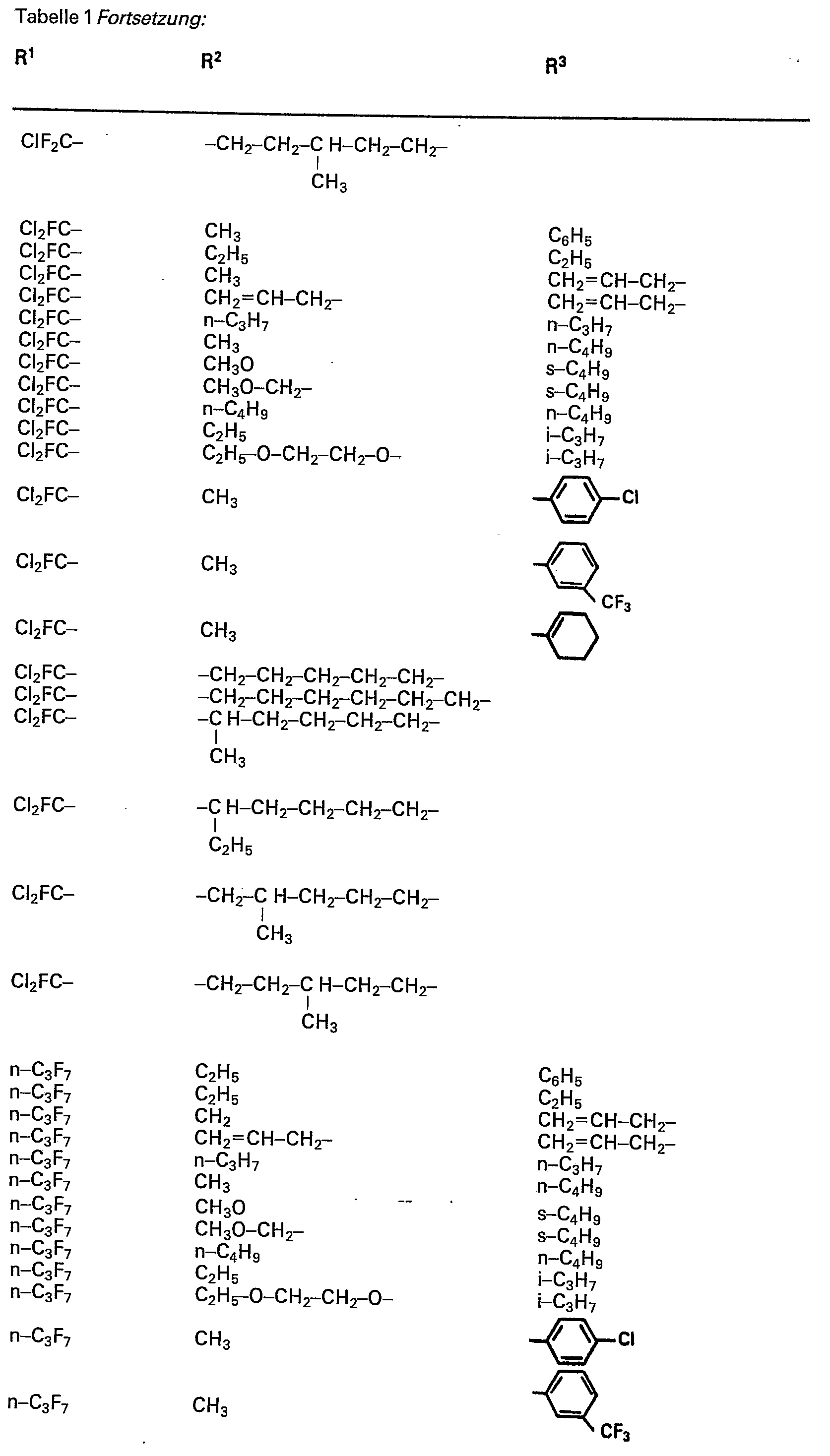

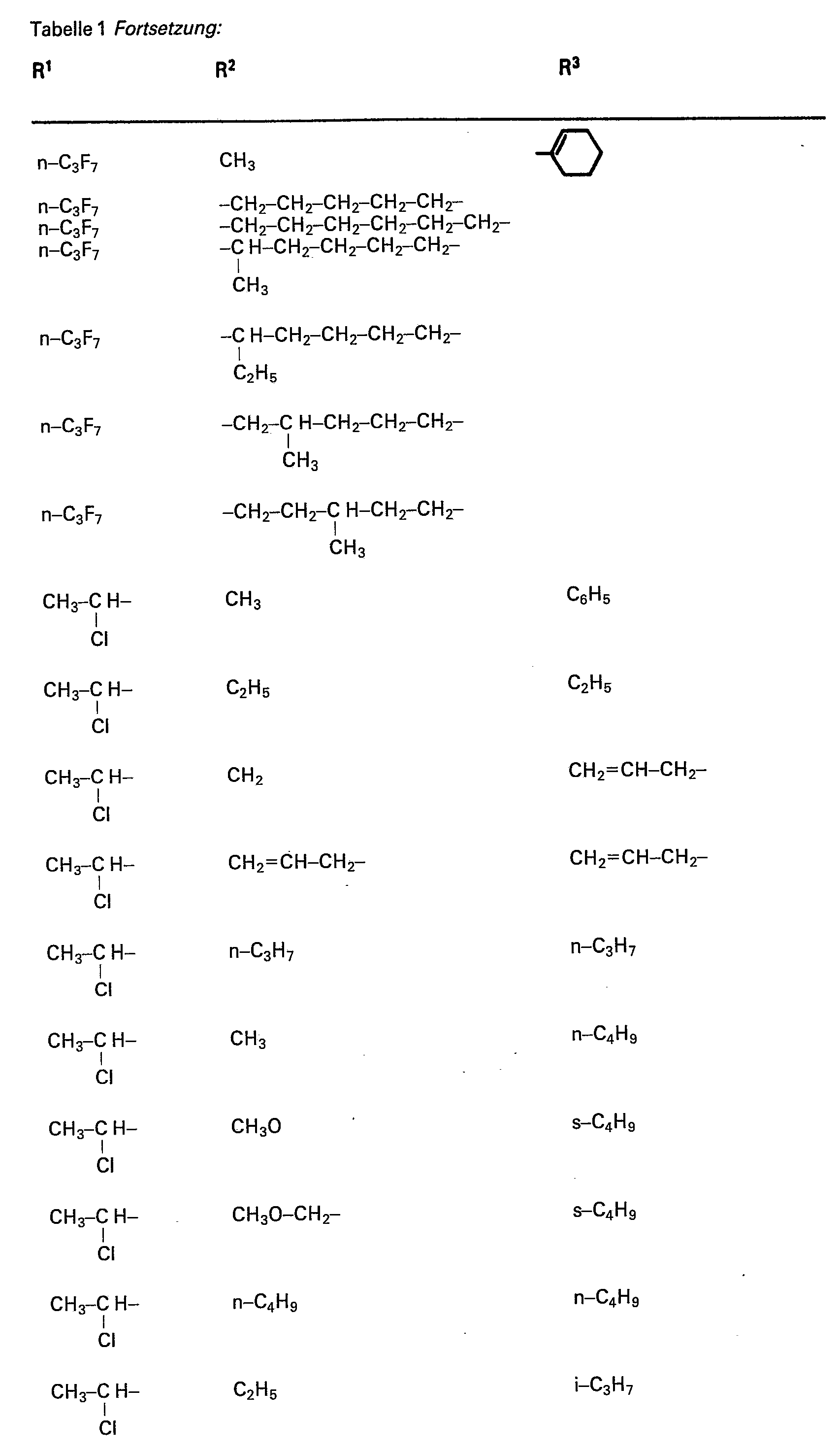

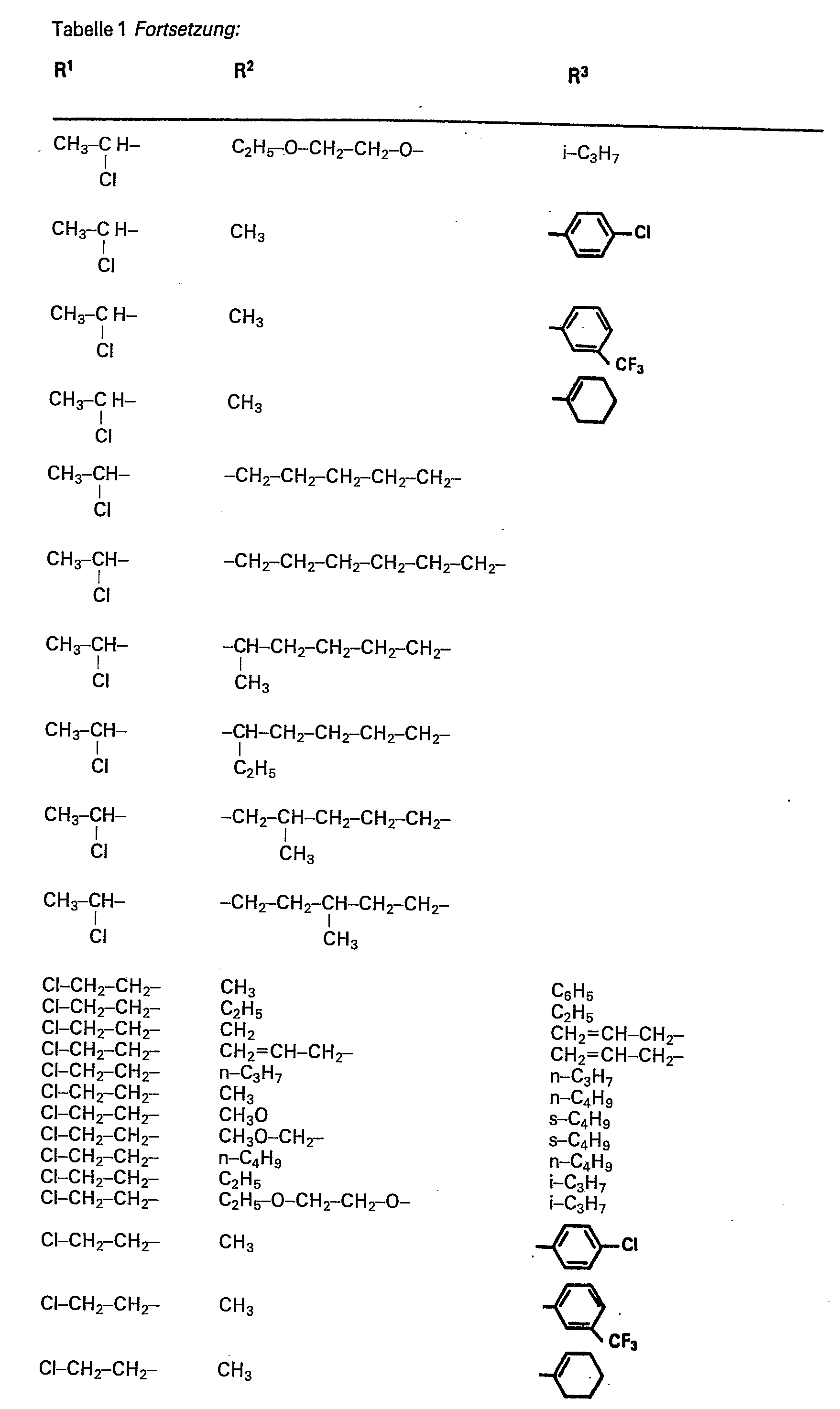

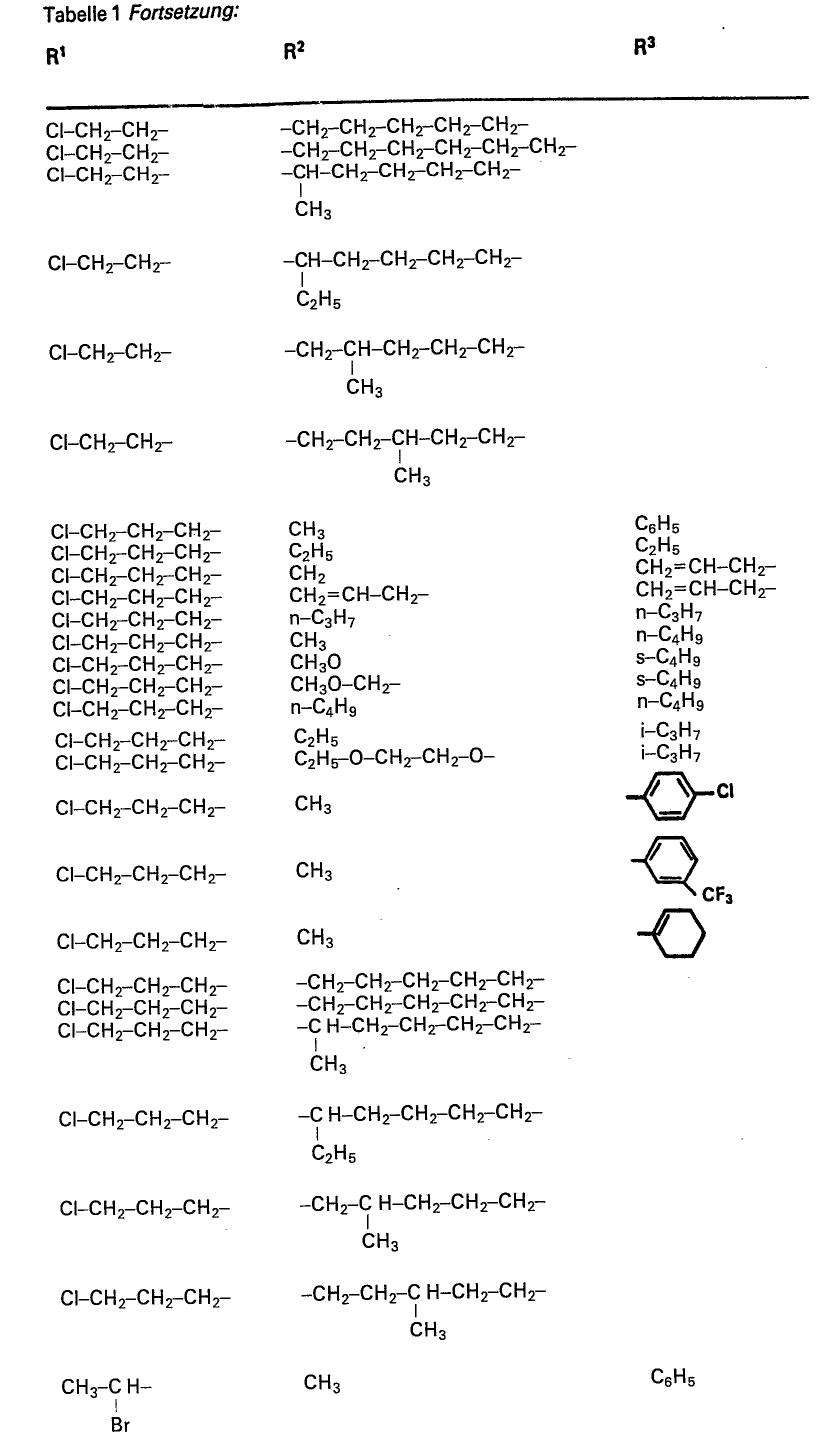

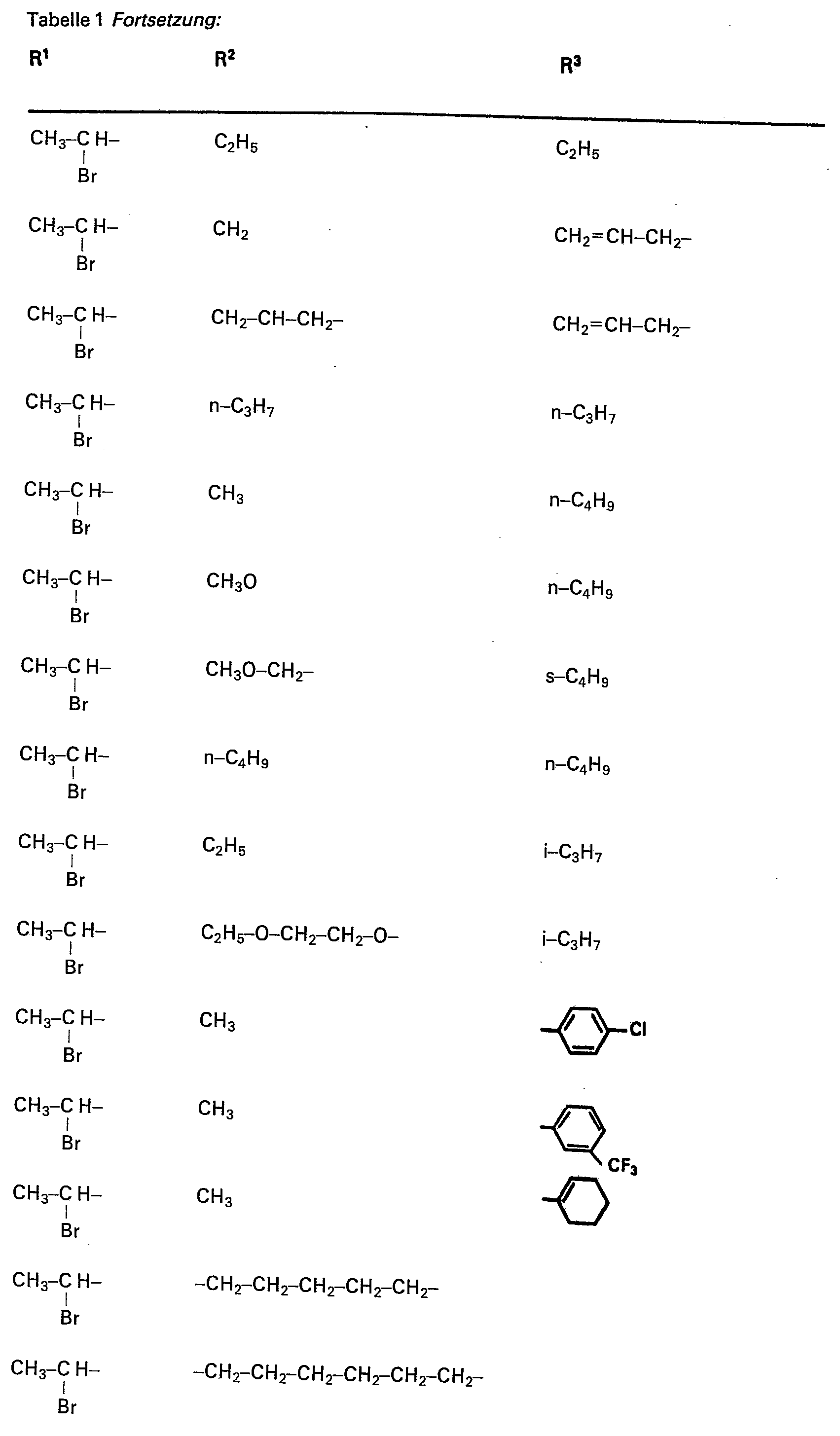

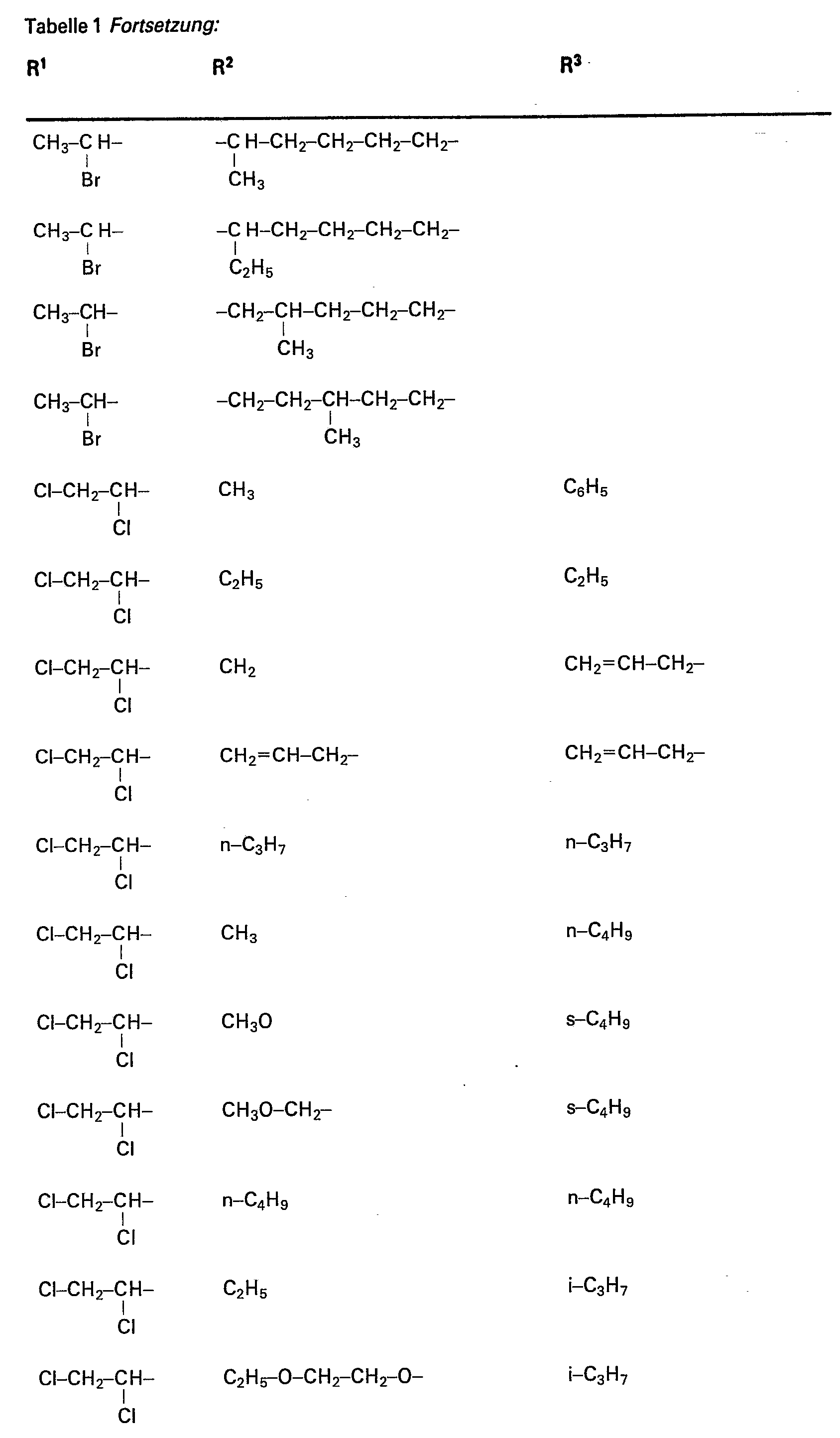

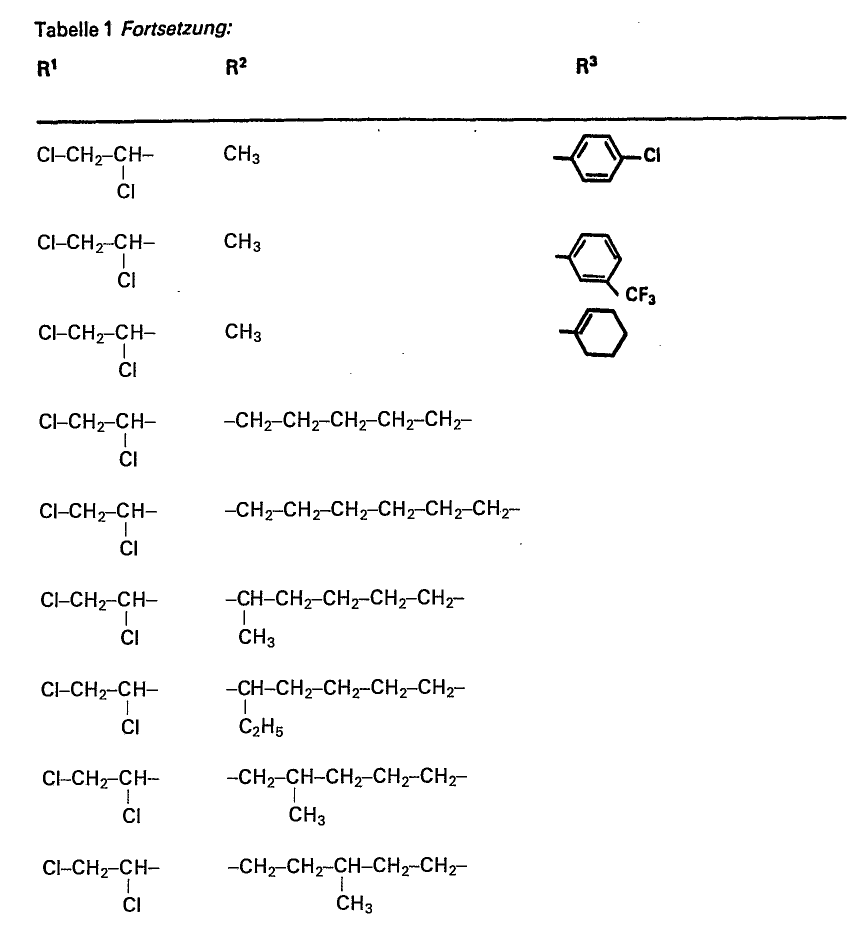

Verwendet man als Ausgangsstoffe beispielsweise 2-Chlor-5-trichlormethyl-1,3,4-thiadiazol und Hydroxyessigsäurediethylamid, so lässt sich der Reaktionsablauf des erfindungsgemässen Verfahrens (a) durch das folgende Formelschema darstellen:

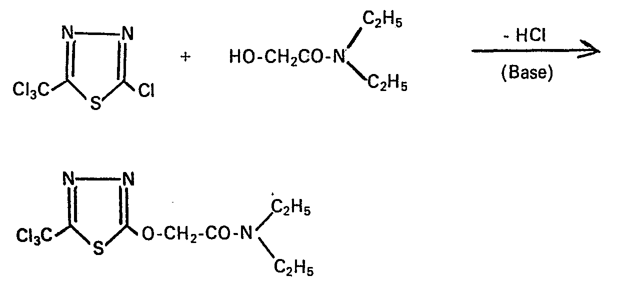

Verwendet man als Ausgangsstoffe beispielsweise Hydrazinthiocarbonsäure-0-(N-methyl-N-phenyl-carbamoylmethylester) und Trichloressigsäureanhydrid, so lässt sich der Reaktionsablauf des erfindungsgemässen Verfahrens (b) durch das folgende Formelschema darstellen:

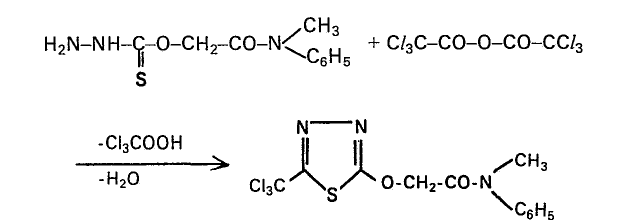

Verwendet man als Ausgangsstoffe beispielsweise N'-Trichloracetylhydrazin-N-thiocarbonsäure-O- (N,N- di- n-propylcarbamoylmethylester) und konzentrierte Schwefelsäure, so lässt sich der Reaktionsablauf des erfindungsgemässen Verfahrens (c) durch das folgende Formelschema darstellen:

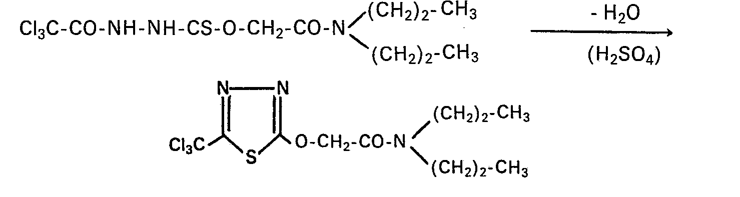

Die als Ausgangsstoffe zur Durchführung des erfindungsgemässen Verfahrens (a) benötigten 5-Halogenalkyl-1,3,4-thiadiazol-Derivate sind durch die Formel (II) allgemein definiert. In dieser Formel (II) steht R¹ vorzugsweise für diejenigen Substituenten, die schon bei der Beschreibung der erfindungsgemässen Stoffe der Formel (I) als bevorzugt für diesen Rest angegeben wurden. A steht vorzugsweise für Halogen oder für Alkylsulfonyl bzw. Aralkylsulfonyl, insbesondere für Chlor, Brom, Methylsulfonyl oder Ethylsulfonyl.

Die 5-Halogen-1,3,4-thiadiazol-Derivate der Formel (II) sind teilweise bekannt (vgl. z.B. Liebigs Ann.Chem. 1980, 1219, DE-OS 18 17069, US-PS 3 562 284).

Die noch nicht bekannten Verbindungen der Formel (II) erhält man in analoger Weise nach prinzipiell bekannten Verfahren (vgl. z.B. DE-OS 32 28 147; J.Chem.Soc. 1949, 1918-1923; J.Chem.Soc. 1949, 3311-3315; US-PS 3 260 588; J.Heterocyclic Chem. Vol. 11 (3), S. 343-345,1974).

Die weiterhin zur Durchführung des erfindungsgemässen Verfahrens (a) als Ausgangsstoffe benötigten Hydroxyacetamide sind durch die Formel (III) allgemein definiert. In dieser Formel (III) stehen R2 und R³ vorzugsweise für diejenigen Reste, die schon bei der Beschreibung der erfindungsgemässen Stoffe der Formel (I) als bevorzugt für diese Substituenten genannt wurden. Die Hydroxyacetamide der Formel (III) sind bekannt (vgl. z.B. EP 18497, EP 29171, DE-OS 30 38 598, DE-OS 31 48 839) oder lassen sich nach prinzipiell bekannten Verfahren in analoger Weise erhalten.

Die zur Durchführung des erfindungsgemässen Verfahrens (b) als Ausgangsstoffe benötigten Hydrazin-thiocarbonsäure-O-carbamoylmethylester sind durch die Formel (IV) allgemein definiert. In dieser Formel (IV) stehen R² und R³ vorzugsweise für diejenigen Reste, die schon bei der Beschreibung der erfindungsgemässen Stoffe der Formel (I) als bevorzugt für diese Substituenten angegeben wurden.

Die Hydrazin-thiocarbonsäure-O-carbamoylmethylester der Formel (IV) sind noch nicht bekannt. Sie sind jedoch Gegenstand einer eigenen parallelen Anmeldung vom gleichen Einreichedatum.

Man erhält sie entweder, wenn man O-Carbamoylmethyl- S-carboxymethyl-dithiocarbonate der Formel (VII), 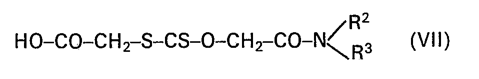 in welcher
R² und R³ die oben angegebene Bedeutung haben, mit Hydrazinhydrat gegebenenfalls in Gegenwart eines Verdünnungsmittels, wie beispielsweise Wasser, und gegebenenfalls in Gegenwart eines Säurebindemittels, wie beispielsweise Natriumhydrogencarbonat, bei Temperaturen zwischen -20 °C und +50 °C umsetzt, oder wenn man Hydroxyacetamide der Formel (III), 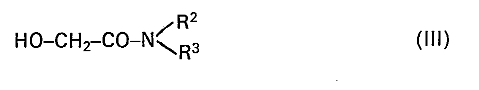 in welcher
R² und R³ die oben angegebene Bedeutung haben, nacheinander in einem «Eintopfverfahren» zunächst mit Schwefelkohlenstoff in Gegenwart einer Base, wie z.B. Alkalimetallhydroxid, dann mit einem Chloressigsäure-Alkalisalz, wie beispielsweise Chloressigsäurenatriumsalz, und zuletzt mit Hydrazinhydrat gegebenenfalls in Gegenwart eines Verdünnungsmittels, wie beispielsweise Wasser, bei Temperaturen zwischen 0 °C und +60 °C umsetzt.

Die O-Carbamoylmethyl-S-carboxymethyl-dithiocarbonate der Formel (VII) sind ebenfalls noch nicht bekannt.

Man erhält sie, wenn man Hydroxyacetamide der Formel (III), 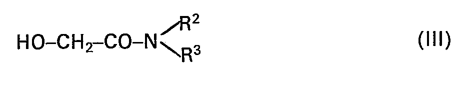 in welcher
R² und R³ die oben angegebene Bedeutung haben, nacheinander in einem «Eintopfverfahren» zunächst mit Schwefelkohlenstoff in Gegenwart einer Base, wie beispielsweise Kaliumhydroxid, dann mit einem Chloressigsäure-Alkalisalz, wie beispielsweise Chloressigsäurenatriumsalz, und zuletzt mit einer Säure, wie beispielsweise Salzsäure, gegebenenfalls in Gegenwart eines Verdünnungsmittels, wie beispielsweise Wasser, bei Temperaturen zwischen 0 °C und +60 °C umsetzt.

Die weiterhin zur Durchführung des erfindungsgemässen Verfahrens (b) als Ausgangsstoffe benötigten Halogencarbonsäureanhydride sind durch die Formel (V) allgemein definiert. In dieser Formel (V) steht R¹ vorzugsweise für diejenigen Reste, die schon bei der Beschreibung der erfindungsgemässen Stoffe der Formel (I) als bevorzugt für diesen Substituenten genannt wurden.

Die Halogencarbonsäureanhydride der Formel (V) sind allgemein bekannte Verbindungen der organischen Chemie.

Die zur Durchführung des erfindungsgemässen Verfahrens (c) als Ausgangsstoffe benötigten N'-Acyl-hydrazin-N-thiocarbonsäure-O-carbamoylmethylester sind durch die Formel (VI) allgemein definiert. In dieser Formel (VI) stehen R^{l}, R² und R³ vorzugsweise für diejenigen Reste, die schon bei der Beschreibung der erfindungsgemässen Stoffe der Formel (I) als bevorzugt für diese Substituenten genannt wurden.

Die N'-Acyl-hydrazin-N-thiocarbonsäure-O-carbamoylmethylester der Formel (VI) sind noch nicht bekannt. Sie sind jedoch Gegenstand einer eigenen parallelen Anmeldung vom gleichen Einreichedatum.

Man erhält sie, wenn man entweder Hydrazin-N-thiocarbonsäure-O-carbamoylmethylester der allgemeinen Formel (IV), 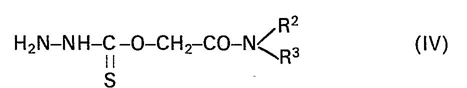 in welcher
R² und R³ die oben angegebene Bedeutung haben, mit Acylierungsmitteln der Formel (VIII), 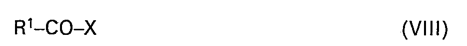
in welcher
R¹ die oben angegebene Bedeutung hat und X für Halogen oder Alkoxy steht, insbesondere für Chlor, Brom, Methoxy oder Ethoxy steht,
gegebenenfalls in Gegenwart eines Verdünnungsmittels, wie beispielsweise Dimethylformamid und gegebenenfalls in Gegenwart eines Säurebindemittels, wie beispielsweise Pyridin, bei Temperaturen zwischen -20 °C und +60 °C umsetzt, oder wenn man

O-Carbamoylmethyl-S-carboxymethyl-dithiocarbonate der Formel (VII), 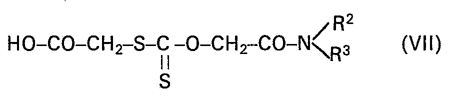 in welcher
R² und R³ die oben angegebene Bedeutung haben, mit N-Acylhydrazinen der Formel (IX), 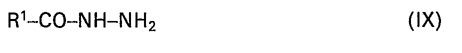
in welcher
R¹ die oben angegebene Bedeutung hat, gegebenenfalls in Gegenwart eines Verdünnungsmittels, wie beispielsweise Wasser, und gegebenenfalls in Gegenwart eines Säurebindemittels, wie beispielsweise Natriumhydrogencarbonat, bei Temperaturen zwischen -20 °C und +50 °C umsetzt, oder wenn man

Hydroxyacetamide der Formel (III) 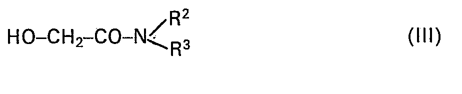 in welcher
R² und R³ die oben angegebene Bedeutung haben, nacheinander in einem «Eintopfverfahren» zunächst mit Schwefelkohlenstoff in Gegenwart einer Base, wie beispielsweise Kaliumhydroxid, dann mit einem Chloressigsäure-Alkalisalz, wie beispielsweise Chloressigsäurenatriumsalz, und zuletzt mit einem N-Acylhydrazin der Formel (IX), 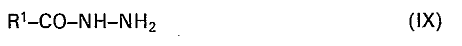
in welcher
R' die oben angegebene Bedeutung hat, gegebenenfalls in Gegenwart eines Verdünnungsmittels, wie beispielsweise Wasser, bei Temperaturen zwischen -20 °C und +60 °C umsetzt.

Acylierungsmittel der Formel (VIII) sind allgemein bekannte Verbindungen der organischen Chemie.

Die N-Acylhydrazine der Formel (IX) sind ebenfalls allgemein bekannte Verbindungen der organischen Chemie oder lassen sich nach prinzipiell bekannten Verfahren in allgemein üblicher Weise durch Acylierung von Hydrazin erhalten (vgl. z.B. C. Ferri, «Reaktionen der organischen Synthse», Thieme Verlag Stuttgart 1978, S. 562, 563).

Als Verdünnungsmittel für das erfindungsgemässe Verfahren (a) kommen organische oder anorganische Lösungsmittel infrage. Bevorzugt sind Kohlenwasserstoffe, wie Toluol oder Cyclohexan, Halogenkohlenwasserstoffe, wie Methylenchlorid, Chloroform, Dichlorethan oder Chlorbenzol, Ketone, wie Aceton oder Methylisobutylketon, Ether, wie Diethylether, Diisopropylether oder Methyl-t-butylether, Alkohole, wie Methanol, Ethanol oder Isopropanol, Amide, wie Dimethylformamid oder Dimethylacetamid, Sulfoxide, wie Dimethylsulfoxid, Wasser oder wässrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemässe Verfahren (a) wird bevorzugt unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z.B. Natrium-, Kalium-, Magnesium- und Calciumhydroxid und/oder Carbonate, wie z.B. Natrium-, Kalium-, Magnesium-und Calciumcarbonat verwendet.

Der Zusatz von 0,01 bis 10 Gewichtsprozent (bezogen auf eingesetztes Hydroxyacetamid der Formel (111) eines Phasentransferkatalysators mag sich in einigen Fällen als vorteilhaft erwiesen. Als Beispiele für solche Katalysatoren seien genannt:

Tetrabutylammoniumchlorid, Tetrabutylammoniumbromid, Tributyl-methylphophoniumbromid, Trimethyl-C₁₃/C₁₅-alkylammoniumchlorid, Dibenzyl-dimethyl-ammonium-methylsulfat, Dimethyl- C_{12/}C₁₄-alkyl-benzylammoniumchlorid, Tetrabutyl-ammoniumhydroxid, 18-Krone-6, Triethylbenzylammoniumchlorid, Trimethylbenzylammoniumchlorid, Tetraethylammoniumbromid, Tetraethylammoniumchlorid.

Die Reaktionstemperaturen können bei dem erfindungsgemässen Verfahren (a) innerhalb eines grösseren Bereiches variiert werden. Sie liegen im allgemeinen zwischen -50 °C und+100 °C.

Das erfindungsgemässe Verfahren (a) wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemässen Verfahrens (a) setzt man pro Mol 5-Halogenalkyl-1,3,4-thiadiazol-Derivat der Formel (11) im allgemeinen 0,1 bis 10 Mol, vorzugsweise 0,8 bis 1,2 Mol an Hydroxyacetamid der Formel (111) und 0,5 bis 10 Mol, vorzugsweise 0,5 bis 5 Mol an Base ein. Die Zugabereihenfolge der Reaktanten kann beliebig vertauscht werden, es können auch alle Komponenten gleichzeitig in das Reaktionsgefäss eindosiert werden. Die Reaktionsführung kann kontinuierlich oder diskontinuierlich gestaltet werden. Die Aufarbeitung erfolgt in üblicher Art und Weise.

Als Verdünnungsmittel zur Durchführung des erfindungsgemässen Verfahrens (b) kommen inerte organische Lösungsmittel infrage. Vorzugsweise verwendet man aliphatische oder aromatische, gegebenenfalls halogenierte Kohlenwasserstoffe, wie Benzin, Benzol, Toluol, Xylol, Hexan, Cyclohexan, Dichlormethan, Chloroform, Tetrachlorkohlenstoff oder Chlorbenzol, Ketone, wie Aceton oder Butanon, Nitrile, wie Acetonitril oder Propionitril, Ether, wie Diethylether, Dioxan, Tetrahydrofuran oder Diisopropylether, Ester, wie Essigsäureethylester oder Amide, wie Dimethylformamid oder Hexamethylphosphorsäuretriamid.

Die Reaktionstemperaturen können bei der Durchführung des erfindungsgemässen Verfahrens (b) in einem grösseren Bereich variiert werden. Im allgemeinen arbeitet man zwischen -30 °C und +30 °C, vorzugsweise zwischen -10 °C und +20 °C.

Zur Durchführung des erfindungsgemässen Verfahrens (b) setzt man pro Mol Hydrazinthiocarbonsäure-O-carbamoylmethylester der Formel (IV) im allgemeinen 1 bis 5 Mol, vorzugsweise 1,5 bis 3 Mol, an Halogencarbonsäureanhydrid der Formel (V) ein. Zur Aufarbeitung entfernt man überschüssiges Anhydrid der Formel (V) und Nebenprodukte durch Behandeln mit wässriger Base und isoliert das Reaktionsprodukt der Formel (I) durch Extraktion mit einem geeigneten mit Wasser nicht mischbaren Lösungsmittel. Die Charakterisierung erfolgt mit Hilfe des Brechungsindexes oder Schmelzpunktes..

Zur Durchführung des erfindungsgemässen Verfahrens (c) kommen als Reaktionshilfsmittel prinzipiell alle üblicherweise für derartige Cyclisierungsreaktionen verwendbaren Mineralsäuren infrage. Vorzugsweise verwendet man konzentrierte Schwefelsäure.

Das erfindungsgemässe Verfahren (c) wird vorzugsweise ohne Zusatz eines Verdünnungsmittels durchgeführt.

Die Reaktionstemperaturen können bei der Durchführung des erfindungsgemässen Verfahrens (c) in einem grösseren Bereich variiert werden. Im allgemeinen arbeitet man zwischen -50°C und +50 °C, vorzugsweise zwischen -30 °C und +20 °C.

Zur Durchführung des erfindungsgemässen Verfahrens (c) setzt man pro Mol N'-Acyl-hydrazin-N-thiocarbonsäure-O-carbamoylester der Formel (VI) im allgemeinen 5 bis 50 Mol, vorzugsweise 10 bis 20 Mol an konzentrierter Schwefelsäure ein. Zur Aufarbeitung verdünnt man die Reaktionsmischung mit Wasser und extrahiert das in Wasser unlösliche Produkt der Formel (I) mit einem geeigneten organischen Lösungsmittel. Die Charakterisierung erfolgt mit Hilfe des Brechungsindexes oder des Schmelzpunktes.

Die erfindungsgemässen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemässen Wirkstoffe der Formel (I) neben einer besonders guten allgemeinen herbiziden Wirksamkeit auch eine deutlich verbesserte Kulturpflanzenselektivität in wichtigen Kulturen und können insbesondere zur Bekämpfung von Unkräutern in Rüben, Sojabohnen, Baumwolle und Getreide eingesetzt werden.

Ausserdem weisen die erfindungsgemässen Wirkstoffe eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei lassen sich die erfindungsgemässen Stoffe mit besonders gutem Erfolg zur Bekämpfung von Getreide- oder Reiskrankheiten, wie beispielsweise gegen die Erreger Pyricularia oryzae, Puccinia recondita, Cochliobolus sativus oder Pyrenophora teres, aber auch gegen Oomyceten oder gegen den Erreger des Apfelschorfes (Venturia inaequalis) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methy)-6-phenyi-1,2,4-triazin-5(4H)-on zur Unkrautbekämpfung in Zuckerrüben und 4-Ami- no-6- (1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Tauchen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Verwendung als Herbizide können die erfindungsgemässen Wirkstoffe sowohl vor als auch nach dem Auflaufen der Pflanzen appliziert werden.

Sie können auch vor der Saat in den Boden eigearbeitet werden.

Die angewandte Wirkstoffmenge kann bei der Verwendung als Herbizide in einem grösseren Bereiche schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 20 kg pro ha.

Bei der Verwendung als Fungizide können die Wirkstoffkonzentrationen in den Anwendungsformen in einem grösseren Bereich variiert werden. Sie liegen bei der Behandlung von Pflanzenteilen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemässen Wirkstoffe geht aus den nachfolgenden Beispielen hervor.

Herstellungsbeispiele:

### Beispiel 1

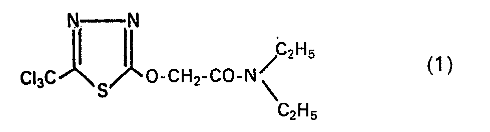

### (Verfahren (a))

Zu einer Lösung von 6,5 g (0,05 Mol) Hydroxyessigsäurediethylamid und 13,8 g (0,05 Mol) 2-Chlor-5-trichlormethyl-1,3,4-thiadiazol in 100 ml Toluol tropft man bei -10°C unter Rühren langsam eine Lösung von 2,4 g (0,06 Mol) Natriumhydroxid in 4 ml Wasser. Nach beendeter Zugabe rührt man weitere 12 Stunden bei 0 °C bis 5 °C. Zur Aufarbeitung wäscht man die organische Phase mit Wasser neutral, engt im Vakuum ein und verreibt den Rückstand bei 0 °C mit Petrolether. Der so erhaltene Feststoff wird abgesaugt und getrocknet.

Man erhält 7 g (42% der Theorie) an N,N-Diethyl-5-trichlormethyl-thiadiazol-2-yloxyacet- amid vom Schmelzpunkt 42 °C.

### Beispiel 2

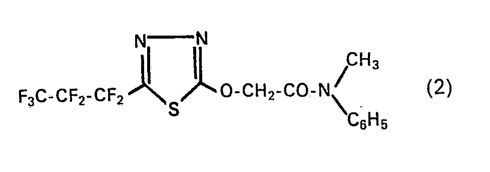

(Verfahren (a)) 175.9

Zu einer Suspension von 3,8 kaliumcarbonat und 0,5 g Tetrabutylammoniumbromid in 50 ml Aceton gibt man bei 20 °C 8,3 g (0,025 Mol) 2-Methylsulfonyl-5-heptafluor-n-propyl-1, 3, - 4-thiadiazol und 4,3 g Hydroxyessigsäure-N-methylanilid. Nach beendeter Zugabe rührt man 20 Stunden bei Raumtemperatur, destilliert das Lösungsmittel ab, nimmt den Rückstand in Wasser auf und extrahiert mit 100 ml Ligroin. Die vereinigten organischen Phasen werden im Vakuum vom Lösungsmittel befreit.

Man erhält 9,1 g (87% der Theorie) an 5-Heptafluor-n-propyl- 1,3,4-thiadiazol- 2-yloxyessigsäure-N-methylanilid als Öl vom Brechungsindex n2⁰ 1,4662.

Herstellung des Ausgangsproduktes (zu Beispiel 2):

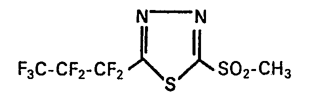

In eine Lösung von 35 g (0,11 Mol) 2-Hepta- fluor-n-propyl-5-methylthio-1, 3, 4-thiadiazol in 280 ml Essigsäure und 70 ml Wasser leitet man bei 5 °C bis 10 °C solange Chlor ein, bis ein gelber Feststoff ausfällt. Das Reaktionsgemisch wird weitere 30 Minuten bei 30 °C nachgerührt und dann mit Chloroform extrahiert. Die vereinigten Chloroformphasen werden mit Wasser gewaschen, über Natriumsulfat getrocknet und im Vakuum eingeengt.

Man erhält 26 g (71 % der Theorie) an 5-Heptafluor-n-propyl- 2-methylsulfonyl- 1,3,4-thiadiazol vom Schmelzpunkt 62 °C

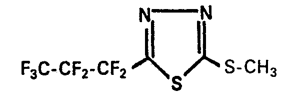

In eine Lösung von 13,8 g (0,1 Mol) Kaliumcarbonat in 20 ml Wasser gibt man bei 20 °C 43 g (0,2 Mol) Perfluorbuttersäure, rührt 15 Minuten bei Raumtemperatur, setzt 200 ml Toluol zu und destilliert das Wasser ab. Die so erhaltene Suspension wird mit 24,2 g (0,2 Mol) Hydrazindithiocarbonsäuremethylester und danach bei 50 °C bis 60 °C mit 39 g (0,25 Mol) Phosphoroxychlorid versetzt. Nach beendeter Zugabe rührt man weitere 2 Stunden bei 70 °C bis 80 °C, giesst die erhaltene Reaktionsmischung auf Eiswasser, trennt die organische Phase ab, wäscht mit Wasser neutral, trocknet über Natriumsulfat und entfernt das Lösungsmittel im Vakuum.

Man erhält 49 g (82% der Theorie) an 5-Hep- tafluor-n-propyl-2-methylthio-1,3,4-thiadiazol als gelbes Öl.

### Beispiel 3

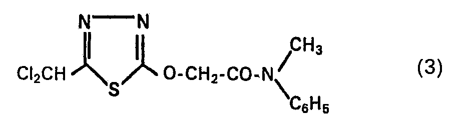

### (Verfahren (b))

Zu einer Mischung aus 11,95 g (0,05 Mol) Hydrazin-thiocarbonsäure-0-(N-methyl-N-phenylcarbamoyl-methylester) und 125 ml Diethylether gibt man bei 0 °C bis 5 °C 28,8 g (0,12 Mol) Dichloressigsäureanhydrid. Anschliessend wird das Reaktionsgemisch 16 Stunden bei Raumtemperatur nachgerührt, mit 20 ml Wasser und dann mit 50 ml gesättigter NatriumhydrogencarbonatLösung geschüttelt, über Natriumsulfat getrocknet und im Vakuum eingedampft. Den Rückstand kristallisiert man aus Isopropanol um.

Man erhält so 7,2 g (44% der Theorie) (5-Dichlormethyl- 1,3,4-thiadiazol-2-yl)-oxyes- sigsäure-N-methylanilid in Form beiger Kristalle mit dem Schmelzpunkt 114 °C.

### Herstellung des Ausgangsproduktes:

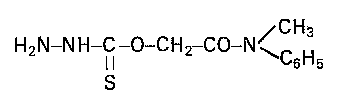

Zu einer Lösung von 11,2 g (0,2 Mol) Kaliumhydroxid in 40 ml Wasser gibt man bei 10 °C erst 33 g (0,2 Mol) Gylkolsäure-N-methylanilid, dann 15,2 g (0,2 Mol) Schwefelkohlenstoff. Man rührt 10 Minuten bei 10 °C bis 15 °C nach und versetzt die dabei entstehende Suspension mit 23,2 g (0,2 Mol) Chloressigsäure-Natriumsalz. Die Temperatur des Reaktionsgemisches steigt auf ca. 38 °C an. Nach 1 Stunde tropft man unter Kühlung bei 5 °C bis 10 °C 10 g (0,2 Mol) Hydrazinhydrat zu, versetzt das Gemisch mit 100 ml Eiswasser und extrahiert 3 mal mit je 50 ml Chloroform.

Nach Abdampfen des Lösungsmittels erhält man 43,2 g (90% der Theorie) Hydrazinthio- carbonsäure-0-(N-methyl-N-phenylcarbamoyl- methyl-ester) in Form hellgrauer Kristalle vom Schmelzpunkt 113°C.

### Beispiel 4

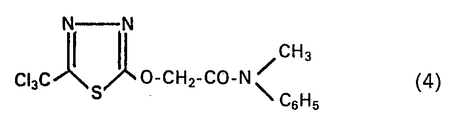

### (Verfahren (c))

11,5g (0,03 Mol) N'-Trichloracetyl-hydrazin-N-thiocarbonsäure-O-(N-methyl-N-phenyl-carbamoyl-methylester) werden bei 0 °C bis 10 °C zu 55 g konzentrierter Schwefelsäure gegeben. Man rührt das Gemisch 2 Stunden bei 0°C bis 10°C nach, giesst es auf 200 g Eiswasser und extrahiert zweimal mit je 50 ml Diethylether. Die organischen Phasen werden erst mit 20 ml Wasser, dann mit gesättigter Natriumhydrogencarbonat-Lösung geschüttelt, über Natriumsulfat getrocknet und im Vakuum eingedampft. Den Rückstand kristallisiert man aus Isopropanol um.

Man erhält auf diese Weise 3,9 g (36% der Theorie) (5-Trichlormethyl-1,3,4-thiadiazol-2-yl)-oxyessigsäure-N-methylanilid in Form farbloser Kristalle vom Schmelzpunkt 100 °C.

### Herstellung des Ausgangsproduktes:

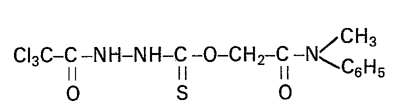

Eine Mischung aus 11,95 g (0,05 Mol) Hydra- zinthiocarbonsäure-0-(N-methyl-N-phenyl-carbamoylmethyl-ester) und 25 ml N,N-Dimethylformamid versetzt man bei 0 °C bis 5 °C erst mit 4 g (0,05 Mol) Pyridin, dann mit 9,1 g (0,05 Mol) Trichloracetylchlorid. Das Gemisch wird 12 Stunde bei 0 °C bis 5 °C nachgerührt und dann mit 100 ml Eiswasser versetzt. Nach Kristallisation saugt man das ausgefallene Reaktionsprodukt ab.

Man erhält so 14,8 g (77% der Theorie) an N'-Trichloracetyl- hydrazin- N-thiocarbonsäure-O-(N-methyl-N-phenylcarbamoylmethyl-ester) in Form eines farblosen Pulvers vom Schmelzpunkt 104 °C (Zersetzung).

In entsprechender Weise und gemäss den allgemeinen Angaben zur Herstellung erhält man die folgenden 5-Halogenalkyl-1,3,4-thiadiazol-2-yloxyacetamide der allgemeinen Formel (I):

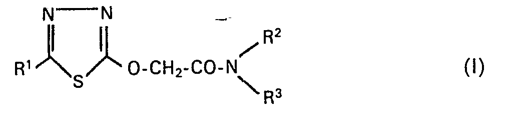

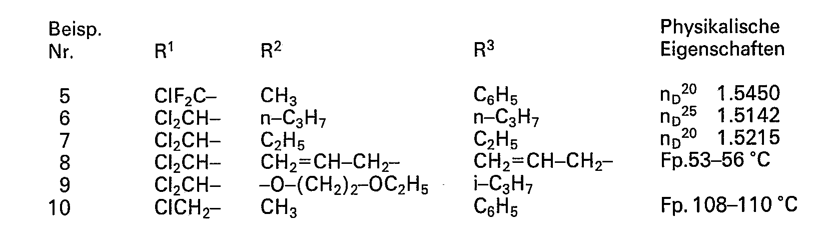

Herstellung weiterer Ausgangsprodukte:

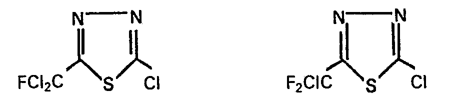

In einem Rührautoklaven aus Edelstahl mit Rückflusskühler und Druckregelung werden 665 g 2-Chlor-5-trichlormethyl-1,3,4-thiadiazol (Siedepunkt 82-83 °C/0,8 mbar; Fp. 38-40 °C) vorgelegt. Bei einer Temperatur von etwa 0 °C lässt man 2 Itr. wasserfreien Fluorwasserstoff zulaufen und verschliesst dann den Autoklaven. Es wird ein Stickstoff-Schutzdruck von 10 bar aufgedrückt und anschliessend langsam bis auf 160 °C geheizt.

Die Reaktion beginnt bei etwa 130-140 °C, was sich in einem deutlichen Druckanstieg bemerkbar macht. Der entstehende Chlorwasserstoff wird über das Regelventil bei 33 bar laufend entspannt. Sobald die Reaktion abklingt, wird der Druck langsam und kontinuierlich auf 30 bar abgesenkt und die Umsetzung bei diesem Druck unter Rückflussbedingungen für den Fluorwasserstoff zu Ende geführt. Der Autoklav wird dann abgekühlt, entspannt und der überschüssige Fluorwasserstoff wird durch Destillation abgetrennt. Den Rückstand giesst man auf Eiswasser, trennt die organische Phase ab und trocknet sie über Natriumsulfat. Das Rohgemisch hat laut gaschromatographischer Analyse folgende Zusammensetzung:

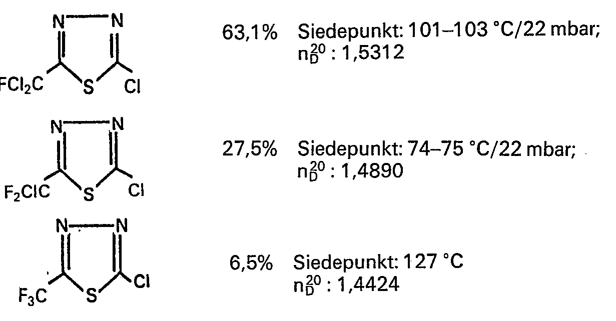

Rest (2,9%): mehrere geringfügige Nebenprodukte.

Durch fraktionierte Destillation werden die Verbindungen 2-Chlor-5-fluordichlormethyl-1,3,4-thiadiazol und 2-Chlor-5-difluorchlormethyl-1,3,4-thiadiazol in reiner Form erhalten. Diese beiden Verbindungen sind neu und sind ebenfalls Gegenstand dieser Erfindung.

### Anwendungsbeispiele:

In den folgenden Anwendungsbeispielen werden die nachstehend aufgeführten Verbindungen als Vergleichssubstanzen herangezogen: 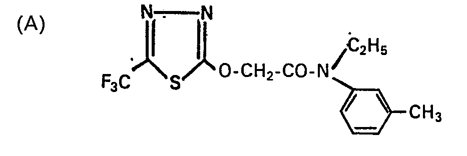
N-Ethyl-N-(3-methylphenyl)-5-trifluormethyl-1,3,4-thiadiazol-2-yloxyacetamid 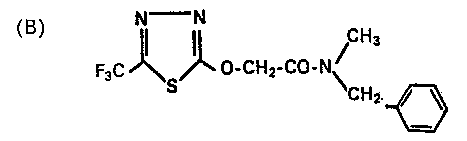
N-Benzyl-N-methyl-5-trifluormethyl-1,3,4-thiadiazol-2-yloxyacetamid (beide bekannt aus D E-OS 3218 482) 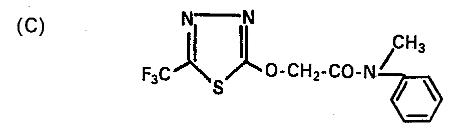
N-Methyl-N-phenyl-5-trifluormethyl-1,3,4-thiadiazol-2-yloxyacetamid (bekannt aus DE-OS 30 04 326)

### Beispiel A

Pre-emergence-Test Lösungsmittel: 5 Gewichtsteile Aceton

Emulgator: 1 Gewichtsteil Alkylarylpolyglykol-ether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneiheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0% =keine Wirkung (wie unbehandelte Kontrolle)
100% =totale Vernichtung

Eine deutliche Überlegenheit in der Wirksamkeit und in der Kulturpflanzenselektivität gegenüber dem Stand der Technik zeigt in diesem Test z.B. die Verbindung gemäss Herstellungsbeispiel 3.

### Beispiel B

Puccinia-Test (Weizen)/protektiv Lösungsmittel: 100 Gewichtsteile Dimethylfomamid

Emulgator: 0,25 Gewichtsteile Alkylarylpolyglykoläther

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1%igen wässrigen Agarlösung inokuliert. Nach Antrocknen besprüht man die Pflanzen mit der Wirkstoffzubereitung taufeucht. Die Pflanzen verbleiben 24 Stunden bei 20 °C und 100% rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt, um die Entwicklung von Rostpustein zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: (3), (4).

### Beispiel C

Pyricularia-Test (Reis)/protektiv Lösungsmittel: 12,5 Gewichtsteile Aceton

Emulgator: 0,3 Gewichtsteile Alkylarylpolyglykoläther

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: (3), (4)

### Beispiel D

Pyricularia-Test (Reis) /systemisch Lösungsmittel: 12,5 Gewichtsteile Aceton

Emulgator: 0,3 Gewichtsteile Alkylarylpolyglykoläther

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25 °C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: (3), (4)

## Patentansprüche

1. 5-Halogenalkyl-1,3,4-thiadiazol-2-yloxy- acetamide der allgemeinen Formel (I), 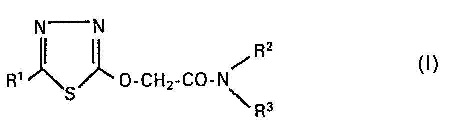 in welcher
R¹ für geradkettiges oder verzweigtes Halogenalkyl mit bis zu 8 Kohlenstoffatomen und bis zu 17 Halogenatomen mit Ausnahme des Trifluor- _{.}methylrestes steht und
R² und R³ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei als Substituenten insbesondere Alkylreste mit 1 bis 4 Kohlenstoffatomen infrage kommen), für jeweils geradkettiges oder verzweigtes Alkoxy, Alkoxyalkylenoxy und Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten möglich sind: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, sowie Nitro, oder
R² und R³ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen, der bis zu 2 weitere Heteroatome, insbesondere Stickstoff und Sauerstoff enthalten kann, wobei als Substituenten möglich sind: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

2. 5- Halogenalkyl-1 ,3,4-thiadiazol-2-yloxy- acetamide der allgemeinen Formel (I) gemäss Anspruch 1, dadurch gekennzeichnet, dass darin
R¹ für Chlormethyl, Dichlormethyl, Trichlormethyl, Fluormethyl, Difluormethyl, Chlorfluormethyl, Chlordifluormethyl, Dichlorfluormethyl, 1-Chlorethyl, 2-Chlorethyl, 1,2-Dichlorethyl, 2,2,2-Trichlorethyl, Pentachlorethyl, Pentafluorethyl, 1-Bromethyl, 2-Bromethyl, 2,2,2-Tribromethyl, 1-Chlorpropyl, 2-Chlorpropyl, 3-Chlorpropyl, 1,2-Dichlorpropyl, 1,2,3-Trichlorpropyl, Heptafluorpropyl, Nonafluorbutyl, Dichlorbutyl, Difluorbutyl, Difluorchlorbutyl oder Dichlorfluorbutyl steht und
R² und R³ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls einbis dreifach, gleich oder verschieden durch Methyl oder Ethyl substituiertes Cycloalkyl bzw. Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen, (insbesondere Fluor, Brom und Chlor), für Benzyl sowie für gegebenenfalls ein- bis dreifach, gleich oder verschieden substituiertes Phenyl stehen, wobei als Substituenten möglich sind: Methyl, Ethyl, Methoxy, Methylthio, Trifluormethyl, Trifluormethoxy, Trifluormethylthio, Fluor, Chlor, Nitro, oder
R² und R³ zusammen mit dem Stickstoffatom, an das sie gebunden sind, für die gegebenenfalls durch Methyl, Ethyl und/oder Phenyl substituierten Heterocyclen 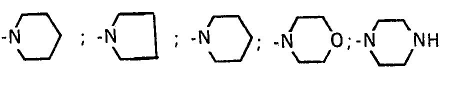 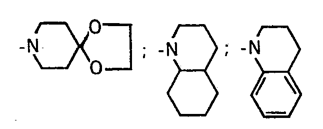
stehen.

3. Verfahren zur Herstellung von 5-Halogenalkyl-1,3,4-thiadiazol-2-yloxyacetamiden der Formel (I) gemäss Anspruch 1, dadurch gekennzeichnet, dass man
(a) 5-Halogenalkyl-1,3,4-thiadiazol-Derivate der Formel (II), 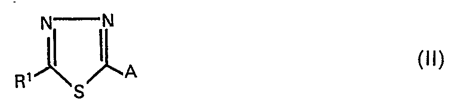 in welcher
R¹ die in Anspruch 1 angegebene Bedeutung hat und
A für eine elektronenanzeihende Abgangsgruppe steht,
mit Hydroxyacetamiden der Formel (III), 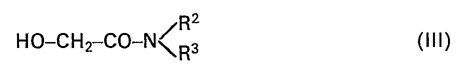 in welcher
R² und R³ die in Anspruch 1 angegebene Bedeutung haben, gegebenenfalls in Gegenwart eines Verdünnungsmittels und gegebenenfalls in Gegenwart eines Säurebindemittels sowie gegebenenfalls in Gegenwart eines Phasentransferkatalysators umsetzt, oder dass man
(b) Hydrazin-thiocarbonsäure-O-carbamoylmethylester der Formel (IV), 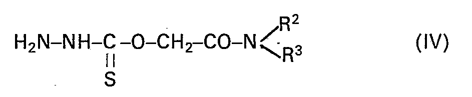 in welcher
R² und R³ die in Anspruch 1 angegebene Bedeutunghaben, mit Halogencarbonsäureanhydriden der Formel (V) 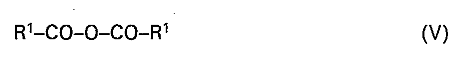
in welcher
R¹ die in Anspruch 1 angegebene Bedeutung hat, gegebenenfalls in Gegenwart eines Verdünnungsmittels umsetzt, oder dass man
(c) N'-Acyl-hydrazin-N-thiocarbonsäure-0-carbamoylmethylester der Formel (VI), 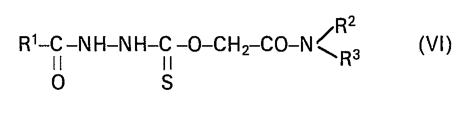 in welcher
R¹, R² und R³ die in Anspruch 1 angegebene Bedeutung haben, mit konzentrierten Mineralsäuren cyclisiert.

4. Herbizide Mittel, gekennzeichnet durch einen Gehalt an mindestens einem 5-Halogenalkyl-1,3,4-thiadiazol-2-yloxyacetamid der allgemeinen Formel (I) gemäss Anspruch 1.

5. Verwendung von 5-Halogenalkyl-1,3,4-thiadiazol-2-yloxyacetamiden der allgemeinen Formel (I) gemäss Anspruch 1 zur Bekämpfung von Unkraut.

6. Fungizide Mittel, gekennzeichnet durch einen Gehalt an mindestens einem 5-Halogenalkyl-1,3,4-thiadiazol-2-yloxyacetamid der allgemeinen Formel (I) gemäss Anspruch 1.

7. Verwendung von 5-Halogenalkyl-1,3,4-thiadiazol-2-yloxyacetamiden der allgemeinen Formel (I) gemäss Anspruch 1 zur Bekämpfung von unerwünschten Mikroorganismen.

8. Verfahren zur Herstellung von herbiziden bzw. fungiziden Mitteln, dadurch gekennzeichnet, dass man 5-Halogenalkyl-1,3,4-thiadiazol-2-yloxyacetamide der allgemeinen Formel (I) gemäss Anspruch 1 mit Streckmitteln und/oder oberflächenaktiven Mitteln vermischt.

9. 2-Chlor-5-halogenalkyl-1,3,4-thiadiazole der Formeln

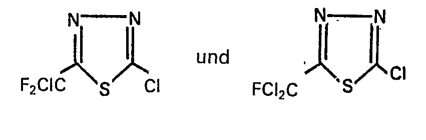

## Revendications

1. 5-halogénalkyl- 1,3,4-thiadiazol- 2-yloxy- acétamides de formule générale (I): 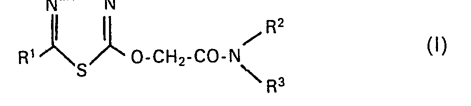 dans laquelle
R¹ représente un radical halogénalkyle à chaîne droite ou ramifiée contenant jusqu'à 8 atomes de carbone et jusqu'à 17 atomes d'halogènes, à l'exception du radical trifluorométhyle, et
R² et R³ représentent chacun indépendamment l'un de l'autre, un atome d'hydrogène, un radical alkyle à chaîne droite ou ramifiée contenant 1 à 8 atomes de carbone, un radical alcynyle et un radical alcényle à chaîne droite ou ramifiée contenant chacun 2 à 8 atomes de carbone, un radical cycloalcényle ou un radical cycloalkyle éventuellement substitué une ou plusieurs fois de manière identique ou différente et contenant chacun 3 à 7 atomes de carbone (en envisageant, comme substituants, en particulier, des radicaux alkyle contenant 1 à 4 atomes de carbone), un radical alcoxyalkyle, un radical alcoxyalkylène-oxy, un radical alcoxy chaque fois à chaîne droite ou ramifiée et contenant chacun 1 à 8 atomes de carbone dans les fractions alkyle ou alkylène individuelles, un radical halogénalkyle contenant 1 à 8 atomes de carbone et 1 à 5 atomes d'halogènes, un radical aralkyle contenant 6 à 10 atomes de carbone dans la fraction aryle et 1 ou 2 atomes de carbone dans la fraction alkyle, ainsi qu'un radical aryle éventuellement substitué une ou plusieurs fois de manière identique ou différente et contenant 6 à 10 atomes de carbone, les substituants possibles étant: les atomes d'halogènes, les radicaux alkylthio, alcoxy ou alkyle à chaîne droite ou ramifiée contenant chacun 1 à 4 atomes de carbone, les radicaux halogénalkyle, les radicaux halogénalcoxy et les radicaux halogénalkylthio contenant chacun 1 ou 2 atomes de carbone et 1 à 5 atomes d'halogènes, de même que le radical nitro, ou R² et R³, ensemble avec l'atome d'azote auquel ils sont reliés, représentent un noyau hétérocyclique pentagonal à heptagonal saturé ou insaturé, éventuellement substitué une ou plusieurs fois de manière identique ou différente et pouvant contenir jusqu'à deux autres hétéro-atomes, en particulier, un atome d'azote et un atome d'oxygène, les substituants possibles étant: les radicaux alkyle à chaîne droite ou ramifiée contenant 1 à 6 atomes de carbone, également sous forme d'un système à noyaux condensés, un radical aryle contenant 6 à 10 atomes de carbone, également sous forme d'un système à noyau condensé, ou un radical dioxyalkylène contenant 2 ou 3 atomes de carbone.

2. 5-halogénalkyl- 1,3,4-thiadiazol- 2-yloxy- acétamides de formule générale (I) selon la revendication 1, caractérisés en ce que, dans cette formule:
R¹ représente le radical chlorométhyle, le radical dichlorométhyle, le radical trichlorométhyle, le radical fluorométhyle, le radical difluorométhyle, le radical chlorofluorométhyle, le radical chlorodi- fluorométhyle, le radical dichlorofluorométhyle, le radical 1-chloréthyle, le radical 2-chloréthyle, le radical 1,2-dichloréthyle, le radical 2,2,2-trichlor- éthyle, le radical pentachloréthyle, le radical pen- tafluoréthyle, le radical 1-brométhyle, le radical 2-brométhyle, le radical 2,2,2-tribrométhyle, le radical 1-chloropropyle, le radical 2-chloropropyle, le radical 3-chloropropyle, le radical 1,2-dichloropropyle, le radical 1,2,3-trichloro- propyle, le radical heptafluoropropyle, le radical nonafluorobutyle, le radical dichlorobutyle, le radical difluorobutyle, le radical difluorochloro- butyle ou le radical dichlorofluorobutyle, et
R² et R³ représentent chacun indépendamment l'un de l'autre un atome d'hydrogène, un radical alkyle à chaîne droite ou ramifiée contenant 1 à 6 atomes de carbone, un radical alcynyle et un radical alcényle à chaîne droite et ramifiée contenant chacun 2 à 6 atomes de carbone, un radical cycloalcényle ou un radical cycloalkyle éventuellement substitué une à trois fois de manière identique ou différente par un radical méthyle ou un radical éthyle et contenant 5 à 7 atomes de carbone, un radical alcoxyalkyle, une radical alcoxyalkylène-oxy ou un radical alcoxy chaque fois à chaîne droite ou ramifiée et contenant chacun 1 à 6 atomes de carbone dans les fractions alkyle individuelles, un radical halogénalkyle contenant 1 à 6 atomes de carbone et 1 à 5 atomes d'halogènes (en particulier, le fluor, le brome et le chlore), un radical benzyle, ainsi qu'un radical phényle éventuellement substitué une à trois fois de manière identique ou différente, les substituants possibles étant: le radical méthyle, le radical éthyle, le radical méthoxy, le radical méthylthio, le radical trifluorométhyle, le radical trifluoromé- thoxy, le radical trifluorométhylthio, l'atome de fluor, l'atome de chlore ou le radical nitro, ou
R² et R³ ensemble avec l'atome d'azote auquel ils sont reliés, représentent les noyaux hétérocycliques éventuellement substitués par le radical méthyle, le radical éthyle et/ou le radical phényle:

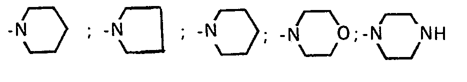

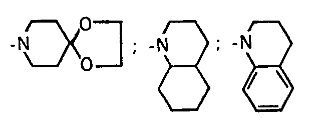

3. Procédé de préparation de 5-halogénalkyl-1,3,4-thiadiazol-2-yloxy-acétamides de formule (I) selon la revendication 1, caractérisé en ce que:
a) on fait réagir des dérivés de 5-halogénalkyl-1,3,4-thiadiazoles de formule (I I): 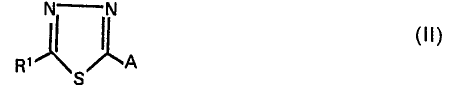 dans laquelle
R¹ a la signification indiquée dans la revendication 1, et
A représente un groupe de départ attirant les électrons,
avec des hydroxy-acétamides de formule (III): 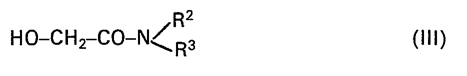 dans laquelle
R² et R³ ont les significations indiquées dans la revendication 1, éventuellement en présence d'un diluant et éventuellement en présence d' un agent fixateur d'acide, ainsi qu'éventuellement en présence d'un catalyseur de transfert de phases, ou
b) on fait réagir des esters O-carbamoyl-méthyliques d'acides hydrazine-thiocarboxyliques de formule (IV): 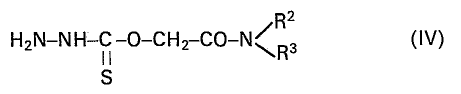 dans laquelle
R² et R³ ont les significations indiquées dans la revendication 1, avec des anhydrides d'acides ha- logénocarboxyliques de formule (V) : 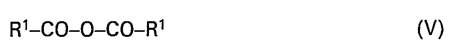 dans laquelle
R¹ a la signification indiquée dans la revendication 1, éventuellement en présence d' un diluant, ou
c) on cyclise des esters O-carbamoyl-méthyliques d'acides N'-acyl-hydrazine-N-thiocarboxyliques de formule (VI): 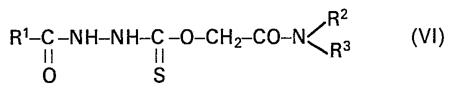 dans laquelle
R', R² et R³ ont les significations indiquées dans la revendication 1, avec des acides minéraux concentrés.

4. Agents herbicides, caractérisés en ce qu'ils contiennent au moins un 5-halogénalkyl-1,3,4-thiadiazol-2-yloxy-acétamide de formule générale (I) selon la revendication 1.

5. Utilisation de 5-halogénalkyl-1,3,4-thiadiazol-2-yloxy-acétamides de formule générale (I) selon la revendication 1 pour combattre les plantes adventices.

6. Agents fongicides, caractérisés en ce qu'ils contiennent au moins un 5-halogénalkyl-1,3,4-thiadiazol-2-yloxy-acétamide de formule générale (I) selon la revendication 1.

7. Utilisation de 5-halogénalkyl-1,3,4-thiadiazol-2-yloxy-acétamides de formule générale (I) selon la revendication 1 pour combattre les micro-organismes indésirables.

8. Procédé de préparation d'agents herbicides ou fongicides, caractérisé en ce qu'on mélange des 5-halogénalkyl- 1,3,4-thiadiazol- 2-yloxy- acétamides de formule générale (I) selon la revendication 1 avec des diluants et/ou des agents ten- sio-actifs.

9. 2-chloro-5-halogénalkyl-1,3,4-thiadiazoles répondant aux formules:

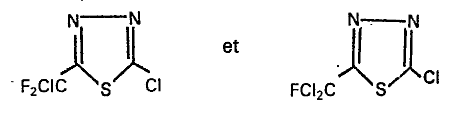

## Claims

1. 5-Halogenoalkyl-1,3,4-thiadiazol-2-yloxy- acetamides of the general formula (1) 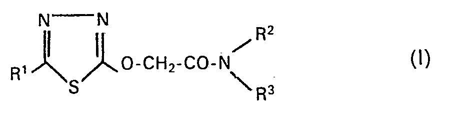 in which
R¹ represents straight-chain or branched halogenoalkyl with up to 8 carbon atoms and up to 17 halogen atoms, with the exception of the trifluoromethyl radical, and
R² and R³ independently of one another represent hydrogen, straight-chain or branched alkyl with 1 to 8 carbon atoms, straight-chain or branched alkenyl or alkinyl with in each case 2 to 8 carbon atoms, cycloalkyl or cycloalkenyl, with in each case 3 to 7 carbon atoms and in each case optionally mono- or polysubstituted by identical or different substituents (possible substituents being, in particular, alkyl radicals with 1 to 4 carbon atoms), in each case straight-chain or branched alkoxy, alkoxyalkylenoxy or alkoxyalkyl with in each case 1 to 8 carbon atoms in the individual alkyl or alkylene parts, halogenoalkyl with 1 to 8 carbon atoms and 1 to 5 halogen atoms, aralkyl with 6 to 10 carbon atoms in the aryl part and 1 or 2 carbon atoms in the alkyl part, or aryl which has 6 to 10 carbon atoms and is optionally mono- or polysubstituted by identical or different substituents, possible substituents being: halogen, straight-chain or branched alkyl, alkoxy and alkylthio with in each case 1 to 4 carbon atoms, halogenoalkyl, halogenoalkoxy and halogenoal- kylthio with in each case 1 or 2 carbon atoms and 1 to 5 halogen atoms and nitro, or
R² and R³, together with the nitrogen atom to which they are bonded, represent a saturated or unsaturated 5-membered to 7-membered heterocyclic radical which is optionally mono- or polysubstituted by identical or different substituents and can contain up to 2 further hetero-atoms, in particular nitrogen and oxygen, possible substituents being: straight-chain or branched alkyl with 1 to 6 carbon atoms, also in the form of a fused-on ring system, aryl with 6 to 10 carbon atoms, also in the form of a fused-on ring system, and dioxyalkylene with 2 or 3 carbon atoms.

2. 5-Halogenoalkyl-1,3,4-thiadiazol-2-yloxy- acetamides of the general formula (I) according to Claim 1, characterized in that, in this formula,
R' represents chloromethyl, dichloromethyl, trichloromethyl, fluoromethyl, difluoromethyl, chlorofluoromethyl, chlorodifluoromethyl, dichlorofluoromethyl, 1-chloroethyl, 2-chloroethyl, 1,2-dichloroethyl, 2,2,2-trichloroethyl, penta- chloroethyl, pentafluoroethyl, 1-bromoethyl, 2-bromoethyl, 2,2,2-tribromoethyl, 1 -chioropro- pyl, 2-chloropropyl, 3-chloropropyl, 1,2-dichloropropyl, 1,2,3-trichloropropyl, heptafluoropro- pyl, nonafluorobutyl, dichlorobutyl, difluorobu- tyl, difluorochlorobutyl or dichlorofluorobutyl and R² and R³ independently of one another represent hydrogen, straight, chain or branched alkyl with 1 to 6 carbon atoms, straight-chain or branched alkenyl or alkinyl with in each case 2 to 6 carbon atoms, cycloalkyl or cycloalkenyl which has 5 to 7 carbon atoms and is optionally mono-, di- or trisubstituted by identical or different substituents from the group comprising methyl and ethyl, in each case straight-chain or branched alkoxy, alkoxyalkylenoxy or alkoxyalkyl with in each case 1 to 6 carbon atoms in the individual alkyl parts, halogenoalkyl with 1 to 6 carbon atoms and 1 to 5 halogen atoms (in particular fluorine, bromine and chlorine), benzyl, or phenyl which is optionally mono-, di- or trisubstituted by identical or different substituents, possible substituents being: methyl, ethyl, methoxy, methylthio, trifluoromethyl, trifluoromethoxy, trifluorome- thylthio, fluorine, chlorine and nitro, or
R² and R³, together with the nitrogen atom to which they are bonded, represent the following heterocyclic radicals which are optionally substituted by methyl, ethyl and/or phenyl

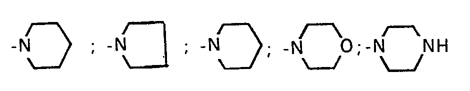

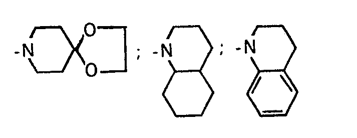

3. Process for the preparation of 5-halogenoalkyl-1,3,4-thiadiazol-2-yloxyacetamides of the formula (I) according to Claim 1, characterized in that (a) 5-halogenoalkyl-1,3,4-thiadiazole derivatives of the formula (II) 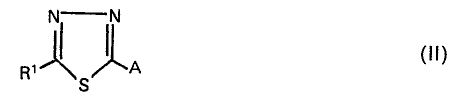 in which
R¹ has the meaning given in Claim 1 and A represents an electron-withdrawing leaving group, are reacted with hydroxyacetamides of the formula (III) 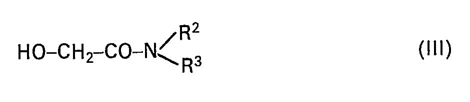 in which
R² and R³ have the meaning given in Claim 1, if appropriate in the presence of a diluent and if appropriate in the presence of an acid-binding agent, and if appropriate in the presence of a phase transfer catalyst, or in that
(b) hydrazine-thiocarboxylic acid O-carbamoylmethyl esters of the formula (IV) 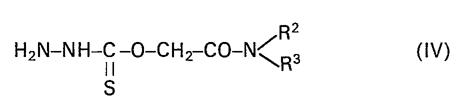 in which
R² and R³ have the meaning given in Claim 1, are reacted with halogenocarboxylic acid anhydrides of the formula (V) 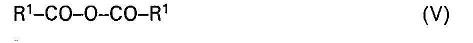 in which
R¹ has the meaning given in Claim 1, if appropriate in the presence of a diluent, or in that
(c) N'-acyl-hydrazine-N-thiocarboxylic acid O-carbamoyl methyl esters of the formula (VI) 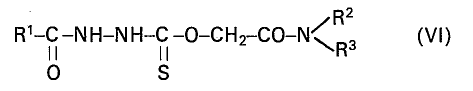 in which
R¹, R² and R³ have the meaning given in Claim 1, are cyclized with concentrated mineral acids.

4. Herbicidal agents, characterized in that they contain at least one 5-halogenoalkyl-1,3,4-thiadiazol-2-yloxyacetamide of the general formula (I) according to Claim 1.

5. Use of 5-halogenoalkyl-1,3,4-thiadiazol-2- yloxacetamides of the general formula (I) according to Claim 1 for combating weeds.

6. Fungicidal agents, characterized in that they contain at least one 5-halogenoalkyl-1,3,4-thiadiazol-2-yloxyacetamide of the general formula (I) according to Claim 1.

7. Use of 5-halogenoalkyl-1,3,4-thiadiazol-2-yloxyacetamides of the general formula (I) according to Claim 1 for combating undesirable microorganisms.

8. Process for the preparation of herbicidal or fungicidal agents, characterized in that 5-halogenoalkyl-1,3,4-thiadiazol-2-yloxyacetamides of the general formula (I) according to Claim 1 are mixed with extenders and/or surface-active agents.

9. 2-Chloro-5-halogenoalkyl-1,3,4-thiadiazoles of the formulae
